# EUROPÄISCHE PATENTSCHRIFT

(11) **EP 1 125 130 B1**
(45) Veröffentlichungstag und Bekanntmachung des Hinweises auf die Patenterteilung: **26.07.2006**
(21) Anmeldenummer: 99971515.4
(22) Anmeldetag: 29.10.1999
(51) Int. Cl.: G01N 33/569, C07K 16/12

(54) **NACHWEIS VON SÄURE-RESISTENTEN MIKROORGANISMEN IM STUHL**
DETECTION OF ACID-RESISTANT MICRO-ORGANISMS IN A STOOL
DETERMINATION DE LA PRESENCE DE MICRO-ORGANISMES RESISTANT AUX ACIDES DANS DES SELLES

(30) Priorität: 29.10.1998 EP 98120517; 06.11.1998 EP 98120687
(43) Veröffentlichungstag der Anmeldung: 22.08.2001
(73) Patentinhaber: DakoCytomation Denmark A/S, 2600 Glostrup (DK)
(72) Erfinder: REITER, Christian, D-85757 Karlsfeld (DE); CULLMANN, Gerhard, D-81371 München (DE); FRIEDRICHS, Ulrike, D-04317 Leipzig (DE); HEPPNER, Petra, D-82049 Pullach (DE); LAKNER, Meret, D-80689 München (DE); RINGEIS, Achim, D-82166 Gräfelfing (DE)
(74) Vertreter: Eisenführ, Speiser & Partner
(86) Internationale Anmeldenummer: PCT/EP1999/008212
(87) Internationale Veröffentlichungsnummer: WO 2000/026671

(56) Entgegenhaltungen:
- EP-A- 0 806 667
- WO-A-92/08485
- WO-A-96/34624
- WO-A-98/04918
- WO-A-98/24885
- YAMAGUCHI H; OSAKI T; TAGUCHI H; HANAWA T; YAMAMOTO T; KAMIYA S: "Production and characterisation of monoclonal antibodies to heat-shock protein 60 of Helicobacter pylori" JOURNAL OF MEDICAL MICROBIOLOGY, Bd. 46, 1997, Seiten 819-824, XP000901295
- NEGRINI R; ZANELLA I; SAVIO A; POIESI C; VERARDI R; GHIELMI S; ALBERTINI A; SANGALETTI O; LAZZARONI M; BIANCHI PORRO G: "Serodiagnosis of Helicobacter pylori-associated gastritis with a monoclonal antibody competitive enzyme-linked immunosorbent assay" SCANDINAVIAN JOURNAL OF GASTROENTEROLOGY, Bd. 27, Nr. 7, Juli 1992 (1992-07), Seiten 599-605, XP000901559

## Beschreibung

Die Erfindung betrifft ein Verfahren zum Nachweis einer Infektion eines Säugers mit einem Säure-resistenten Mikroorganismus, wobei man (a) eine Stuhlprobe des Säugers mit mindestens zwei verschiedenen monoklonalen Antikörpern, Fragmenten oder Derivaten davon oder Aptameren unter Bedingungen inkubiert, die eine Komplexbildung von Antigenen aus dem Säure-resistenten Mikroorganismus mit den Antikörpern, Fragmenten oder Derivaten davon oder den Aptameren erlauben, und wobei (aa) der erste monoklonale Antikörper oder das Fragment oder Derivat davon oder das erste Aptamer ein Epitop des ersten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert; (ab) der zweite monoklonale Antikörper oder das Fragment oder Derivat davon oder das zweite Aptamer ein vom Epitop des ersten Antigens verschiedenes Epitop eines zweiten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert, wobei die Teile der Säuger gemäß (aa) und gemäß (ab) überlappen können und in der Summe im wesentlichen die Gesamtanzahl der infizierten Säuger ausmachen; und (b) die Bildung mindestens eines Antigen-Antikörperkomplexes/Antigen-Aptamerkomlexes gemäß (aa) oder (ab) nachweist.

Vorzugsweise ist der Säure-resistente Mikroorganismus ein Bakterium, insbesondere *Helicobacter pylori, Helicobacter hepaticus* oder *Mycobacterium tuberculosis* oder *Campylobacter jejuni* oder *Campylobacter pylori*. Ferner bevorzugt ist, daß die beiden Epitope Epitope einer Urease und eines Hitzeschockproteins, vorzugsweise von Hsp60, einer Alkylhydroperoxid-Reduktase, vorzugsweise des 26kDa-Proteins, des 20kDa-Proteins (3-Dehydro-Quinase Typ II), des 16,9kDa-Proteins (Neutrophile-aktivierendes Protein) oder des 33,8 kDa-Proteins (Fruktose-Bisphosphat-Aldolase) sind. In einer weiteren bevorzugten Ausführungsform der Erfindung wird die Stuhlprobe mit drei verschiedenen monoklonalen Antikörpern, Fragmenten oder Derivaten davon oder Aptameren untersucht, wobei der erste Antikörper, das Fragment oder Derivat davon oder das erste Aptamer ein Epitop einer Urease und der zweite Antikörper und der dritte Antikörper (oder das Derivat, Fragment oder Aptamer) je ein Epitop von unterschiedlichen vorstehend genannten Proteinen, vorzugsweise Hsp60 (wenn eines der beiden ersten Epitope ein Epitop einer Alkylhydroperoxid-Reduktase ist) oder eine Alkylhydroperoxid-Reduktase spezifisch bindet, wenn eines der beiden ersten Epitope ein Epitop von Hsp60 ist. Ferner betrifft die Erfindung diagnostische und pharmazeutische Zusammensetzungen und Testvorrichtungen, die die vorgenannten Komponenten enthalten sowie diese enthaltende Verpackungen.

Der Nachweis der Infektion eines Säugerorganismus mit einem mikrobiellen Pathogen oder Parasiten kann heute auf verschiedene, invasive wie nicht-invasive, direkte und indirekte Arten geführt werden. Beim Einsatz invasiver Techniken wird die körperliche Integrität des Untersuchten verletzt, z.B. bei der Entnahme einer Biopsie oder von Serum. Nicht-invasive Diagnosetechniken stellen Veränderungen in Parametern fest, die ohne einen Eingriff in den Organismus gemessen werden können. Bevorzugt werden hierzu Körperflüssigkeiten und Ausscheidungen wie Atemluft, Urin, Speichel, Schweiß oder Stuhl beprobt und analysiert. Bei direkten Methoden wird das Vorhandensein des Pathogens oder Parasiten, seiner Bestandteile oder deren Abbauprodukte nachgewiesen, z.B. durch Färbung und mikroskopische Untersuchung oder spezifische enzymatische Reaktionen. Indirekte Verfahren greifen zurück auf den Nachweis von Reaktionen des Wirtsorganismus auf das Pathogen oder den Parasiten, z.B. das Vorhandensein von Antikörpern gegen Antigene des Pathogens im Serum, im Urin oder im Speichel des Wirts. In einem weiteren indirekten Verfahren müssen dem Patienten Indikatorsubstanzen zugeführt werden, die von dem mikrobiellen Pathogen oder Parasiten in spezifischer Weise modifiziert werden, wobei die modifizierte Form in Proben nachgewiesen wird (Atemtest).

Ein Eingriff in den Organismus, z.B. zur Erlangung einer Gewebeprobe, ist für den Organismus in den meisten Fällen belastend und häufig auch mit hohem apparativen Aufwand und einem gesundheitlichen Risiko verbunden. Die vergleichsweise einfache Erfassung von Proben der oben beschriebenen Körperflüssigkeiten und Ausscheidungen machen daher nicht-invasive Techniken zur Methode der Wahl. Da weiterhin nicht jeder Wirt in gleicher Weise auf ein bestimmtes Pathogen oder einen bestimmten Parasiten reagiert, und die Reaktion des Wirts mit Verzögerung einsetzen und zudem auch nach Entfernung des Pathogens oder Parasiten aus dem Organismus persistieren kann, sind direkte Verfahren stets vorzuziehen. Idealerweise wird also eine Diagnose durch den nichtinvasiven, direkten Nachweis des Pathogens oder Parasiten in Körperflüssigkeiten oder Ausscheidungen geführt.

Ein diagnostisches Verfahren sollte darüber hinaus auch auf weitere Gesichtspunkte hin optimiert sein: Hohe Reproduzierbarkeit, Sensitivität und Spezifität, garantierte Verfügbarkeit der zu verwendenden Materialien in konstanter Qualität, geringe Kosten bei Herstellung und Durchführung, und einfache Anwendung unabhängig von aufwendigen Apparaturen sind hier zu berücksichtigende Parameter.

Aus den oben genannten Gründen nehmen Verfahren basierend auf der hohen Selektivität und Bindungsaffinität bestimmter Substanzklassen (z.B. Antikörper, Rezeptoren, Lektine, Aptamere) für molekulare Strukturen, welche so gewählt werden können, daß sie für den jeweils zu bestimmenden Stoff/Mikroorganismus hochspezifisch sind, in der medizinischen Diagnostik einen zunehmend breiten Raum ein. Insbesondere die Möglichkeit der Immobilisierung dieser Substanzen an Festkörperoberflächen sowie der Kopplung mit radioaktiven Nukliden, mit Enzymen, die in Kombination mit geeigneten Substraten Farbreaktionen auslösen, oder mit farbigen Partikeln mit der hochspezifischen Bindungsaffinität (z.B. ELISA = Enzyme linked immunosorbent assay) führte zu der Entwicklung kostengünstiger, einfacher und wenig zeitaufwendiger Nachweisverfahren für körpereigene wie körperfremde Stoffe.

In den Anfangsphasen der Entwicklung dieser Nachweisverfahren fanden ausschließlich polyklonale Antikörper Verwendung. Diese haben jedoch einige, dem Fachmann wohlbekannte Nachteile, so vor allem begrenzte Verfügbarkeit und oft auch Kreuzreaktivitäten. Die Erarbeitung von Verfahren zur Herstellung monoklonaler Antikörper (Köhler & Milstein (1975)), die Fortschritte bei der Isolierung von Rezeptoren und deren gerichtete Expression in zellulären Wirtssystemen, die Entwicklung von Lektinen mit hoher Affinität für bestimmte Kohlenhydrate sowie die Entdeckung, daß Nukleinsäuren (Aptamere) molekulare Strukturen spezifisch binden können, konnten diese Nachteile großenteils beseitigen. Heute lassen sich mit vergleichsweise einfachen Methoden die Spezifität und Sensitivität von Nachweisverfahren optimieren.

Aufgrund der hohen Spezifität eignen sich derartige Verfahren besonders zum Nachweis einzelner, definierter Substanzen wie Haptene, Peptide oder Proteine, vorausgesetzt, das erkannte Strukturelement ist konstant innerhalb der zu untersuchenden Probenpopulation und spezifisch für die nachzuweisende Substanz. Sie sind zudem für eine Messung in Körperflüssigkeiten gut geeignet, und stellen damit eine naheliegende Option für den direkten Nachweis von Pathogenen in dieser Probenmatrix dar. Entsprechend sind im Stand der Technik Verfahren zur Diagnose z.B. von *Entamoeba histolytica* (Haque (1993), J. Infect. Dis. **167:** 247-9), enterohemorrhagischen *Escherichia coli* (EHEC; Park (1996), J. Clin. Microbiol. **34**: 988-990), *Vibrio cholerae* (Hasan (1994), FEMS Microbiol. Lett. **120:** 143-148), Torovirus-ähnlichen Partikeln (Koopmans (1993), J. Clin. Microbiol. **31:** 2738-2744) oder *Taenia saginata* (Machnicka (1996), Appl. Parasitol. **37:** 106-110) aus Stuhl beschrieben. Ein Diagnoseverfahren für Adenovirus-bedingte Gastroenteritis (Hermann (1987), J. Infect. Dis. **155:** 1167-1171) verwendet die Kombination aus zwei, gegen unterschiedliche enterische Adenovirus-Serotypen gerichteten, monoklonalen Antikörpern, um diese Serotypen unbeeinflußt von der Präsenz anderer, nicht enterischer Adenoviren nachweisen zu können. Hierbei gelingt der Nachweis bei Kenntnis des Adenovirus-Serotyps, mit dem ein Patient identifiziert ist, auch mit dem jeweiligen monoklonalen Antikörper allein zuverlässig.

Den oben beschriebenen Pathogenen ist gemeinsam, daß sie im Darm, in der Darmschleimhaut oder im Darmlumen ihres Wirts, in allen Fällen des Menschen, lebens- und vermehrungsfähig sind. Sie besitzen also Mechanismen, die Ihnen das Überleben und die Vermehrung in Anwesenheit der im Darm aktiven Abbau- und Verdauungssysteme erlauben. Damit ist es wahrscheinlich, daß bei der Ausscheidung mit dem Stuhl eine hohe Anzahl intakter oder fast intakter Pathogene bzw. Parasiten abgehen. Mit Nachweisreagenzien, beispielsweise Antikörpern, welche Bestandteile der Pathogene bzw. Parasiten erkennen, können diese im Stuhl oder in aufbereiteten Stuhlproben in der Regel leicht nachgewiesen werden.

Es gibt jedoch eine Anzahl von Pathogenen und Parasiten, die einerseits aufgrund der Beziehungen der von ihnen befallenen Gewebe (z.B. Lunge, Magen, Pankreas, Duodenum, Leber) zum Magen-Darm-Trakt im Stuhl auftreten können, andererseits aber im Darm selbst nicht lebens- und/oder vermehrungsfähig sind. Diese Pathogene und Parasiten werden hier als Säure-resistente Mikroorganismen bezeichnet. Zu diesen Pathogenen und Parasiten gehören beispielsweise *Helicobacter pylori (H. pylori)* und *Helicobacter hepaticus, Mycobacterium tuberculosis* und andere Mycobakterien, *Chlamydia pneumoniae, Legionella pneumophilae, Pneumocystis carinii, Campylobacter jejuni, Campylobacter pylori* und andere. Einige dieser Erreger können z.B. im Sputum nachgewiesen werden, jedoch ist beispielsweise der Nachweis von *Mycobacterium tuberculosis* im Sputum nur während eines kurzen Zeitraums möglich, und zwar nachdem sich eine den Erreger enthaltende Kaverne geöffnet hat. Ein Nachweis wird ferner dadurch erschwert, daß es nicht immer möglich ist, von einem zu Untersuchenden eine Sputumprobe zu erhalten. Dies trifft z.B bei Kleinkindern, verwirrten Patienten oder Tieren zu. Andere Pathogene wie beispielsweise *Legionella pneumophilae* lassen sich anhand von Antigenen, die über die Niere in den Urin gelangen spezifisch nachweisen. Dies gelingt jedoch nur, wenn die im Urin befindliche Menge für den Nachweis ausreicht. Der Nachweis im Stuhl wäre hierzu eine begrüßenswerte Alternative. Die Darmpassage ist bei diesen Organismen jedoch mit einem starken Angriff durch die Verdauungs- und Abbaumechanismen des Säugers und insbesondere der Darmflora verbunden. Für den betrachteten Erreger spezifische molekulare Strukturen können dabei zerstört oder in ihrer Konzentration stark herabgesetzt werden.

Die Degradation der Erreger im Darm hat sich bei Säure-resistenten Bakterien als Problem für einen sicheren Nachweis in Stuhlproben erwiesen. Die Zahl der Keime, die in den Magen eines Infizierten gelangen, ist im Vergleich zu anderen, sich im Darm ansiedelnden Bakterien gering. Zudem müssen Keime und Keimbruchstücke nach Verlassen des Magens einen langen Weg durch den an Proteasen reichen Darm zurücklegen. Diese Umstände haben zur Folge, daß sich nur geringe Mengen intakter Proteine im Stuhl wiederfinden lassen. Dies bedingt auch, daß die für einen ELISA-Test nötige Kombination zweier Epitope auf einem Antigen nicht mehr notwendigerweise wie im nativen Protein gegeben ist, und nah nebeneinander liegende Epitope die größte Wahrscheinlichkeit haben, in einem Nachweis, der zwei Epitope auf demselben Molekül benötigt, ein positives Ergebnis zu zeigen. Die individuell unterschiedliche Verteilung von im Stuhl von Infizierten nachgewiesenen Antigenen weist zusätzlich auf individuelle Eigenschaften in der Prozessierung der Antigene beim Darmdurchgang hin. Ein erster Ansatz, dieses Problem zu verringern, wurde durch den Offenbarungsgehalt der EP-A 0 806 667 bereitgestellt. In dieser Anmeldung wurde gezeigt, daß polyklonale Antikörper mit dem Lysat eines bestimmten *H. pylori*-Stammes induziert werden konnten, die eine breitere Variabilität von Stämmen aus verschiedenen geographischen Regionen erkennen. Allerdings geht aus dieser Anmeldung nicht hervor, welche Antigene durch das Serum erkannt werden. Ferner wurde eine entsprechende Diagnose vermittels monoklonaler Antikörper ausgeschlossen. Angesichts der Tatsache, daß Immunseren trotz aller Standardisierungsbemühungen schwanken können, muß das in der o.g. Anmeldung entwickelte Verfahren für eine breite Anwendung als suboptimal betrachtet werden. Hinzu kommt, daß für die Bereitstellung der polyklonalen Seren immer wieder neue Tiere immunisiert werden müssen. Die entsprechenden Verfahren sind sowohl zeit- als auch kostenintensiv.

R. Negrini et al. beschreiben in SCANDINAVIAN JOURNAL OF GASTROENTEROLOGY, Bd. 27, Nr. 7, Juli 1992 (1992-07), Seiten 599-605, die Verwendung von monoklonalen Antikörpern gegen Helicobacter pylori zur Diagnose einer auf H. pylori zurückzuführenden Gastritis im Serum. In diesem Artikel wird auf die Gefahr von Kreuzreaktionen zwischen H.pylori Antigenen und Antigenen verwandter Stämme hingewiesen. Weiterhin beschreiben H. Yamaguchi et al. in JOURNAL OF MEDICAL MICROBIOLOGY, Bd. 46, (1997), Seiten 819-824 die Herstellung und Charakterisierung spezifischer monoklonaler Antikörper, die gegen das Hitzeschockprotein 60 von H. pylori gerichtet sind.

In WO 96/34624 werden immunogene Zusammensetzungen gegen Helicobacter-Infektionen beschrieben, um gegen Helicobacter gerichtete Antikörper zu erzeugen. Keine der genannten Literaturstellen offenbart jedoch Antikörper, die als geeignet zum Nachweis von H.pylori in einer Stuhlprobe angesehen werden könnten.

Aufgabe der vorliegenden Erfindung war somit, die vorgenannten nachteiligen Verfahren aus dem Stand der Technik zu verbessern und ein leicht standardisierbares sowie kostengünstiges Verfahren zum sicheren Nachweis von Säure-resistenten Mikroorganismen bereitzustellen.

Diese Aufgabe wird durch die in den Ansprüchen gekennzeichneten Ausführungsformen gelöst.

Somit betrifft die Erfindung ein Verfahren zum Nachweis einer Infektion eines Säugers mit einem Säure-resistenten Mikroorganismus, wobei man
(a) eine Stuhlprobe des Säugers mit zwei oder drei verschiedenen monoklonalen Antikörpern, Fragmenten oder Derivaten davon oder Aptameren unter Bedingungen inkubiert, die eine Komplexbildung von Antigenen aus dem Säure-resistenten Mikroorganismus mit den Antikörpern, Fragmenten oder Derivaten davon oder den Aptameren erlauben, und wobei
   (aa) der erste monoklonale Antikörper oder das Fragment oder Derivat davon oder das erste Aptamer ein Epitop des ersten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid, das eine Sequenz des Proteins oder Fragments aufweist, Antikörper produziert;
   (ab) der zweite monoklonale Antikörper oder das Fragment oder Derivat davon oder das zweite Aptamer ein vom Epitop des ersten Antigens verschiedenes Epitop eines zweiten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid, das eine Sequenz des Proteins oder Fragments aufweist, Antikörper produziert;
      wobei die Teile der Säuger gemäß (aa) und gemäß (ab) überlappen können und in der Summe im wesentlichen die Gesamtanzahl der infizierten Säuger ausmachen; und
(b) die Bildung mindestens eines Antigen-Antikörperkomplexes oder Antigen-Aptamerkomplexes gemäß (aa) oder (ab) nachweist.

Der Begriff "Säure-resistenter Mikroorganismus" im Sinne dieser Erfindung umfaßt jeden Mikroorganismus, der aufgrund seiner Eigenschaften/Anpassungsmechanismen an den Wirt den physikalischen und chemischen Einflüssen des Verdauungstrakts widersteht, so daß er durch einen vorzugsweise immunologischen Nachweis oder unter Verwendung von Aptameren detektierbar ist. Beispiele für derartige Säure-resistente Mikroorganismen sind *Helicobacter pylori, Helicobacter hepaticum, Mycobacterium tuberculosis, Mycobacterium pseudotuberculosis, Mycobacterium cansassii, Camplyobacter jejuni* und *Campylobacter pylori*.

Der Begriff "Stuhlprobe des Säugers" bedeutet im Sinne dieser Erfindung jede Stuhlprobe, die für das erfindungsgemäße Nachweisverfahren eingesetzt werden kann. Insbesondere fallen darunter Stuhlproben, die nach an sich bekannten Verfahren für diagnostische Tests aufbereitet worden sind. Die Aufbereitung erfolgt beispielsweise gemäß RIDASCREEN® Entamoeba Enzymimmunoassay (R-Biopharm GmbH, Darmstadt).

"Fragmente" oder "Derivate" von monoklonalen Antikörpern weisen im Sinne dieser Erfindung dieselbe Bindungsspezifität wie die monoklonalen Antikörper auf. Derartige Fragmente oder Derivate können nach üblichen Verfahren hergestellt werden; vgl. z.B. Harlow und Lane "Antibodies, A Laboratory Manual", CSH Press, Cold Spring Harbor, USA, 1988. Beispiele für Fragmente sind Fab-, F(ab')₂ oder Fv-Fragmente. Beispiele für Derivate sind scFv-Fragmente. Derivate können auch chemisch hergestellte Substanzen sein, die dieselben oder verbesserte Bindungseigenschaften wie die Antikörper aufweisen. Solche Substanzen können beispielsweise durch Peptidomimetics oder durch verschiedene Runden von Phage Display und nachfolgende Selektion auf verbesserte Bindungseigenschaften hergestellt werden. Unter Aptameren werden erfindungsgemäß Nukleinsäuren wie RNA, ssDNA (ss = Einzelstrang), modifizierte RNA oder modifizierte ssDNA verstanden, die eine große Vielzahl von Zielsequenzen mit hoher Spezifität und Affinität binden. Der Begriff "Aptamer" ist im Stand der Technik bekannt und definiert beispielsweise in Osborne et al., Curr. Opin. Chem. Biol. 1 (1997), 5-9, oder in Stull und Szoka, Pharm. Res. 12 (1995), 465-483.

Der Begriff "mindestens zwei" ist hinsichtlich der Anzahl von verwendeten Antikörpern etc. nach oben nicht limitiert und bedeutet beispielsweise drei, vier, fünf, sechs, sieben, acht, neun oder zehn Antikörper etc.

"Bedingungen, die eine Komplexbildung erlauben" sind vom Fachmann ohne weiteres einstellbar; s. auch Harlow und Lane, a.a.O., und sind beispielsweise physiologische Bedingungen.

Der Begriff "nach der Darmpassage eine Struktur aufweist, die der nativen Struktur entspricht" bedeutet im Sinne dieser Erfindung, daß das Epitop eines Antigens nach der Darmpassage von einem monoklonalen Antikörper, Derivat oder Fragment davon oder dem Aptamer erkannt wird, der/das gegen dasselbe Antigen/Epitop gewonnen wurde oder an dieses bindet, welches die Darmpassage nicht passiert hat. Mit anderen Worten, das Epitop/Antigen, das vom o.g. Antikörper bzw. Fragmenten oder Derivate davon oder Aptameren spezifisch gebunden wird, hat die Darmpassage hinsichtlich seiner Struktur unbeschadet oder im wesentlichen unbeschadet überstanden und ist nicht degradiert worden. Als Quelle für die native Struktur des Epitops/Antigens kann z.B. ein mit einer French-Press aufgeschlossener Bakterienextrakt, der mit üblichen Verfahren (vgl. z.B. Sambrook et al., "Molecular Cloning, A Laboratory Manual", 2. Auflage 1989, CSH Press, Cold Spring Harbor, USA) weiter aufgereinigt wurde, oder ein Bakterienlysat, das nach Standardverfahren weiter aufgereinigt wurde (z.B. Sambrook et al., a.a.O.), dienen.

Der Begriff "nach der Darmpassage eine Struktur aufweist, die der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert" bedeutet erfindungsgemäß, daß das vom monoklonalen Antikörper, Fragment, Derivat oder Aptamer erkannte Epitop einem Epitop entspricht, das vom Immunsystem eines Säugers, vorzugsweise eines Menschen, präsentiert wird. Die Mechanismen der Antigen-Präsentation sowie Mechanismen, die zur Prozessierung von Antigenen führen und die daraus resultierende Antikörpervielfalt sind im Stand der Technik bekannt und beispielsweise in Janeway und Travers, Immunologie, 2. Auflage 1997, Spektrum Akademischer Verlag GmbH, Heidelberg beschrieben. Diese Epitope können sich von den nativen Epitopen unterscheiden. Der Kontakt des Säugers mit den Mikroorganismen oder den Proteinen oder Fragmenten oder den synthetischen Peptiden kann durch natürliche Infektion (außer bei den synthetischen Peptiden) oder durch Immunisierung erfolgen. Für die Immunisierung können auch Extrakte, Lysate, synthetische Peptide etc. des Mikroorganismus/Proteins herangezogen werden. Geeignete Immunisierungsschemata sind im Stand der Technik bekannt und beispielsweise beschrieben in Harlow und Lane, a.a.O. Geeignete Antikörper können beispielsweise auch durch Immunisierung und/oder Screening auf Surrogate wie synthetische Peptide, rekombinant hergestellte Proteine, Extrakte, Lysate oder partiell verdaute Proteine gewonnen werden.

Der Begriff "spezifisch bindet" bedeutet erfindungsgemäß, daß der monoklonale Antikörper, das Fragment oder Derivat davon oder Aptamer keine oder im wesentlichen keine Reaktivität mit anderen Epitopen in Proben nicht infizierter Säuger aufweist.

Der Begriff "wobei die Teile der Säuger gemäß (aa) und gemäß (ab) überlappen können und in der Summe im wesentlichen die Gesamtanzahl der infizierten Säuger ausmachen" bedeutet, daß mindestens eines, gegebenenfalls aber auch zwei oder mehr Epitope/Antigene im Stuhl auftreten, die von den monoklonalen Antikörpern, Fragmenten oder Derivaten oder den Aptameren spezifisch gebunden werden und daß die monoklonalen Antikörper, Fragmente oder Derivate oder die Aptamere mindestens ein Epitop eines Antigens bei im wesentlichen jedem infizierten Säuger detektieren. Der Begriff "im wesentlichen die Gesamtzahl" bedeutet dabei, daß die Epitope und damit eine entsprechende Infektion mit dem Mikroorganismus bei mehr als 70%, vorzugsweise mindestens 75%, stärker bevorzugt mehr als 85%, besonders bevorzugt mehr als 90% und am meisten bevorzugt mehr als 95% der Betroffenen erfaßt werden kann. Idealerweise werden Infektionen bei 100% der Betroffenen nachgewiesen.

Der Begriff "Antigen-Antikörperkomplex" im Sinne dieser Erfindung umfaßt nicht nur Komplexe, die das Antigen mit dem nativen Antikörper eingeht, sondern auch solche, die es mit dessen Fragmenten oder Derivaten eingeht.

Überraschenderweise wurde erfindungsgemäß gefunden, daß mit einer limitierten Anzahl von monoklonalen Antikörpern, Fragmenten oder Derivaten davon oder Aptameren, die mindestens zwei verschiedene Epitope von verschiedenen Antigenen Säure-resistenter Mikroorganismen spezifisch binden, eine relativ sichere Diagnose der Infektion mit diesen Bakterien/Pathogenen durchgeführt werden kann. So können in dieser Ausführungsform der Erfindung Antigene einer aufbereiteten Stuhlprobe bespielsweise an eine Festphase gebunden werden und das infizierende Agens mit der limitierten Anzahl von in markierter Form vorliegenden monoklonalen Antikörpern, Fragmenten oder Derivaten davon oder Aptameren nachgewiesen werden. Sofern das nach der Darmpassage vorliegende Antigen (noch) in di- oder multimerer Form vorliegt, können die gleichen monoklonalen Antikörper, Fragmente oder Derivate davon oder Aptamere sowohl als Fänger wie auch als Detektor eingesetzt werden. Diese Ausführungsform umfaßt auch die Möglichkeit, daß ein Epitop mehrmals auf derselben Untereinheit vorkommt. Die Erfindung schließt Ausführungsformen mit ein, in denen weitere Epitope, die die vorgenannten Eigenschaften aufweisen, von weiteren monoklonalen Antikörpern oder Fragmenten oder Derivaten davon oder von weiteren Aptameren erkannt werden. Auch kann ein Antigen von verschiedenen monoklonalen Antikörpern oder Aptameren, die dasselbe oder unterschiedliche Epitope des Antigens erkennen, nachgewiesen werden. So können beispielsweise drei monoklonale Antikörper oder drei Aptamere drei unterschiedliche Epitope auf zwei Proteinfragmenten erkennen. Letztere Ausführungsformen sind dazu geeignet, die Sicherheit bei der Stellung der Diagnose noch weiter zu erhöhen. Im Falle höhergeordneter Strukturen, z.B. von Di- oder Multimeren, kann der gleiche monoklonale Antikörper auch mehrere Epitope im selben multimeren Protein im erfindungsgemäßen Verfahren binden. Beispiele derartiger Ausführungsformen werden nachfolgend genauer beschrieben.

Umfaßt von der Erfindung sind Ausführungsformen, bei denen nur monoklonale Antikörper oder Fragmente oder Derivate davon oder nur Aptamere eingesetzt werden, wie auch Ausführungsformen, bei denen in einem Test unterschiedliche Arten von Nachweisreagenzien eingesetzt werden. So ist es möglich, daß ein erster monoklonaler Antikörper mit einem zweiten Antikörperderivat oder ein erstes Aptamer mit einem zweiten Antikörperfragment eingesetzt wird, um nur zwei Beispiele zu nennen. Insofern bezeichnen die Begriffe "erste" und "zweite" das erste und zweite Nachweisreagens. Gemeint ist dabei nicht, daß immer zwei Antikörper, Derivate oder Fragmente davon oder immer zwei Aptamere eingesetzt werden.

Die erfindungsgemäßen Ergebnisse sind vor allem deshalb überraschend, da der Stand der Technik hiervon weggelehrt hatte. So wurde beispielsweise bei *H. pylori* gefunden, daß Hauptantigene in ELISA-Tests nicht die gewünschte Spezifität und Sensitivität aufweisen; vgl. Newell et al., Serodiag. Immunother. Infect. Dis. 3 (1989), 1-6. Darüber hinaus lehrt die EP-A 0 806 667, daß ein sicherer Nachweis von *H. pylori*-Infektionen mit monoklonalen Antikörpern aufgrund der genetischen Variabilität der *H. pylori*-Stämme nicht möglich sei.

Das erfindungsgemäße Verfahren ist gegenüber dem erwähnten Stand der Technik insbesondere deshalb von Vorteil, da mit lediglich zwei monoklonalen Antikörpern oder Fragmenten oder Derivaten davon oder Aptameren eine relativ sichere Diagnose ermöglicht wird. Vorzugsweise werden für den Nachweis, beispielsweise im ELISA Pärchen von Antikörpern, Fragmenten, Derivaten davon oder Aptameren eingesetzt, wobei die beiden Antikörper des Pärchens dasselbe oder unterschiedliche Epitope auf demselben Antigen binden. Beispielsweise bildet *H. pylori*-Urease multimere Strukturen aus mehreren gleichen wie auch unterschiedlichen Untereinheiten aus. Im ELISA oder anderen Assays können somit die gleichen Antikörper, Fragmente oder Derivate davon oder Aptamere als Fängerantikörper/aptamere wie auch als Detektionsantikörper/aptamere eingesetzt werden. Zum einen gewährt die Verwendung von monoklonalen Antikörpern, Fragmenten oder Derivaten davon oder von Aptameren einen leicht zu haltenden Standard bei der Zuverlässigkeit des Diagnoseverfahrens, was ein großer Vorteil im Vergleich zu bisher bekannten und für diesen Zweck eingeführten Diagnoseverfahren ist. Weiterhin entfällt das beispielsweise im Verfahren der EP-A 0 806 667 notwendige immer neue Immunisieren und nachfolgende Testen von Versuchstieren. Ein weiterer Vorteil des erfindungsgemäßen Verfahrens ist seine Ausgestaltung als direktes und nicht-invasives Verfahren, was die einleitend genannten Annehmlichkeiten für den Patienten sowie die Zuverlässigkeit bei der Bestimmung des Krankheitsstadiums erhöht.

In einer bevorzugten Ausführungsform ist der Säure-resistente Mikroorganismus ein Säure-resistentes Bakterium.

Im Stand der Technik ist eine Reihe von Säure-resistenten Bakterien bekannt. In einer besonders bevorzugten Ausführungsform ist das Säure-resistente Bakterium ein Bakterium der Gattung Helicobacter oder der Gattung Mycobacterium oder der Gattung Campylobacter.

In einer anderen besonders bevorzugten Ausführungsform ist das Bakterium ein Bakterium der Spezies *Helicobacter pylori, Helicobacter hepaticum* oder ein Bakterium der Spezies *Mycobacterium tuberculosis* oder ein Bakterium der Spezies *Campylobacterjejuni* oder *Campylobacter pylori.*

In einer weiteren bevorzugten Ausführungsform ist das Epitop des ersten Antigens ein Epitop einer Urease, z.B. einer α-Urease oder einer β-Urease, und das Epitop des zweiten Antigens ein Epitop eines Hitzeschockproteins, einer Alkylhydroperoxid-Reduktase oder des 20kDa-Proteins (3-Dehydro-Quinase Typ II), des 16,9kDa-Proteins (Neutrophile-aktivierendes Protein) oder des 33,8 kDa-Proteins (Fruktose-Bisphosphat-Aldolase).

Überraschenderweise wurde erfindungsgemäß gefunden, daß in einer Population von Säugern, insbesondere von menschlichen Patienten, deren Stuhl auf Infektionen mit Säure-resistenten Bakterien getestet wurde, im wesentlichen alle Mitglieder dieser Population mindestens eines der o.g. Epitope im Stuhl aufwiesen, so daß mit hoher Wahrscheinlichkeit mit einem Satz von nur zwei entsprechenden monoklonalen Antikörpern, Fragmenten oder Derivaten davon oder Aptameren eine relativ sichere Diagnose gestellt werden kann.

Besonders bevorzugt ist, daß die Urease die β-Urease von *H. pylori* ist.

In einer weiteren besonders bevorzugten Ausführungsform ist das Hitzeschockprotein ein Hsp60.

Weiterhin ist besonders bevorzugt, daß es sich bei dem Hsp60 um das Hsp 60 von *H. pylori* handelt.

In einer zusätzlich besonders bevorzugten Ausführungsform ist die Alkylhydroperoxid-Reduktase das 26kDa-Protein von *H. pylori*.

Auch ist bevorzugt, daß die weiteren vorstehend genannten Proteine Proteine von *H. pylori* sind.

In einer weiteren (bevorzugten) Ausführungsform umfaßt das erfindungsmäße Verfahren zum Nachweis einer Infektion eines Säugers mit einem säure-resistenten Mikroorganismus die folgenden Schritte, wobei man (a) eine Stuhlprobe des Säugers mit drei verschiedenen monoklonalen Antikörpern, Fragmenten oder Derivaten davon oder Aptameren unter Bedingungen inkubiert, die eine Komplexbildung von Antigenen aus dem Säure-resistenten Mikroorganismus mit den Antikörpern, Fragmenten oder Derivaten davon oder Aptameren erlauben, und wobei (aa) der erste monoklonale Antikörper oder das Fragment oder Derivat davon oder das erste Aptamer ein Epitop des ersten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert; (ab) der zweite monoklonale Antikörper oder das Fragment oder Derivat davon oder das zweite Aptamer ein vom Epitop des ersten Antigens verschiedenes Epitop eines zweiten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert; und (ac) der dritte monoklonale Antikörper oder das Fragment oder Derivat davon oder das dritte Aptamer ein vom Epitop des ersten und zweiten Antigens verschiedenes Epitop eines dritten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert, wobei die Teile der Säuger gemäß (aa), gemäß (ab) und gemäß (ac) überlappen können und in der Summe im wesentlichen die Gesamtanzahl der infizierten Säuger ausmachen, und die Bildung mindestens eines Antigen-Antikörperkomplexes/Antigen-Aptamerkomlexes gemäß (aa), (ab) oder (ac) nachweist.

Hinsichtlich der Definitionen und bevorzugten Ausführungsformen dieses Verfahrens wird auf die vorstehend erläuterten Ausführungsformen verwiesen. Die Erläuterungen zur Kombination verschiedenartiger Nachweisreagenzien beim Einsatz von mindestens zwei Nachweisreagenzien gelten hier entsprechend für drei Nachweisregenzien. So kann ein erster monoklonaler Antikörper mit einem zweiten Fragment und einem dritten Aptamer im Test kombiniert werden. Diese Kombination bedeutet selbstverständlich nicht, daß noch zwei weitere Aptamere im Test eingesetzt werden. Der Begriff "dritte" bezieht sich somit auf das dritte Nachweisreagens, unabhängig von dessen möglicher Struktur. Entsprechendes gilt für die Begriffe "erste" und "zweite".

Bevorzugt ist das Epitop des ersten Antigens ein Epitop einer Urease, vorzugsweise der β-Urease von *H. pylori*, das Epitop des zweiten Antigens ein Epitop eines Hitzeschockproteins, vorzugsweise von Hsp60, vorzugsweise aus *H. pylori*, und das Epitop des dritten Antigens ein Epitop einer Alkylhydroperoxid-Reduktase, vorzugsweise des 26kDa-Proteins von *H. pylori*. In anderen bevorzugten Ausführungsformen sind die Epitope des zweiten und des dritten Antigens Epitope des 20kDa-Proteins (3-Dehydro-Quinase Typ II), des 16,9kDa-Proteins (Neutrophile-aktivierendes Protein) oder des 33,8 kDa-Proteins (Fruktose-Bisphosphat-Aldolase), in Kombination mit der Urease als erstem und ggf. dem Hsp60 oder dem 26kDa-Protein als zweiten oder dritten Antigen. In einer weiteren bevorzugten Ausführungsform sind die Epitope des zweiten Antigens Epitope von Hsp60 und die Epitope des dritten Antigens Epitope des 20kDa-Proteins (3-Dehydro-Quinase Typ II), des 16,9kDa-Proteins (Neutrophile-aktivierendes Protein) oder des 33,8 kDa-Proteins (Fruktose-Bisphosphat-Aldolase). Weitere Ausführungsformen unter Nachweis von Kombinationen der vorstehend genannten Proteine bzw. deren Epitope, auch ohne Urease, sind möglich.

Bei Reihenuntersuchungen von Stuhlproben von mit *H. pylori* infizierten Patienten auf verschiedene *H. pylori*-spezifische Proteine (Urease, HSP60, Alkylhydroperoxid-Reduktase) mittels ELISA unter Verwendung monoklonaler Antikörper wurde gefunden, daß die Auffindbarkeit verschiedener Antigene in Stuhlproben verschiedener Patienten sehr inhomogen ist. Weiter fand sich überraschenderweise, daß Kombinationen aus monoklonalen Antikörpern, die teilweise überlappende Epitope erkennen, für den Nachweis dieser Antigene mittels Sandwich-ELISA eine sehr gute Eignung besaßen (siehe Tabelle 3).

Die monoklonalen Antikörper, Fragmente oder Derivate davon oder die Aptamere können erfindungsgemäß lineare oder Konformationsepitope erkennen und spezifisch binden. In einer weiteren bevorzugten Ausführungsform bindet mindestens einer der monoklonalen Antikörper bzw. eines der Fragmente, Derivate oder Aptamere ein Konformationsepitop.

In einer besonders bevorzugten Ausführungsform binden sämtliche monoklonalen Antikörper etc. Konformationsepitope.

Im Epitop-mapping wurden 5 der im Stuhl-ELISA besonders geeigneten murinen mAK (monoklonalen Antikörper) untersucht (Tabelle 3). Überraschenderweise konnte bei drei dieser Antikörper kein lineares Epitop bestimmt werden. Daher wurde angenommen, daß diese mAK Konformationsepitope erkennen. Diese Aussage wird für die anti-β-Urease Antikörper durch die Immunblotergebnisse bestätigt, die zeigen, daß die denaturierte Urease nicht mehr oder nur sehr schwach erkannt wird (siehe Tabelle 2).

Die Erfindung betrifft in einer anderen Ausführungsform ein Verfahren zum Nachweis einer Infektion mit *Helicobacter pylori* im Stuhl eines Säugers, wobei man (a) eine Stuhlprobe mit mindestens zwei verschiedenen monoklonalen Antikörpern, Fragmenten, Derivaten davon oder Aptameren unter Bedingungen inkubiert, die eine Antigen-Antikörper/Antigen-Aptamer-Komplexbildung erlauben, wobei (aa) der erste monoklonale Antikörper, das Fragment, Derivat davon oder das erste Aptamer spezifisch β-Urease oder ein Fragment davon bindet; (ab) der zweite monoklonale Antikörper, das Fragment, Derivat davon oder das zweite Aptamer spezifisch das 26kDa-Antigen oder ein Fragment davon bindet oder spezifisch Hsp60 oder ein Fragment davon bindet; und (b) die Bildung mindestens eines Antigen-Antikörperkomplexes/Antigen-Aptamerkomlexes gemäß (aa) oder (ab) nachweist.

Ferner betrifft die Erfindung ein Verfahren zum Nachweis einer Infektion mit *Helicobacter pylori* im Stuhl eines Säugers, wobei man (a) eine Stuhlprobe mit drei verschiedenen monoklonalen Antikörpern, Fragmenten, Derivaten davon oder Aptameren unter Bedingungen inkubiert, die eine Antigen-Antikörper/Antigen-Aptamer-Komplexbildung erlauben, wobei (aa) der erste monoklonale Antikörper, das Fragment, Derivat davon oder das erste Aptamer spezifisch β-Urease oder ein Fragment davon bindet; (ab) der zweite monoklonale Antikörper, das Fragment, Derivat davon oder das zweite Aptamer spezifisch Hsp60 oder ein Fragment davon bindet; und (ac) der dritte monoklonale Antikörper, das Fragment, Derivat davon oder das dritte Aptamer spezifisch das 26kDa-Antigen oder ein Fragment davon bindet; und (b) die Bildung mindestens eines Antigen-Antikörper/Antigen-AptamerKomplexes gemäß (aa), (ab) oder (ac) nachweist.

Hinsichtlich der verwendeten Definitionen und anwendbaren bevorzugten Ausführungsformen wird auf die vorherigen Erläuterungen hingewiesen.

Die genannten Proteine in den beiden vorstehenden Ausführungsformen sind selbstverständlich *H. pylori*-Proteine.

In einer anderen bevorzugten Ausführungsform ist der Hsp60-spezifische Antikörper der von dem bei der Deutschen Sammlung von Mikroorganismen und Zellkulturen (DSMZ) am 23. Juni 1998 nach den Statuten des Budapester Vertrages unter der Hinterlegungsnummer DSM ACC2356 hinterlegten Hybridom HP16m/2A5-E6-E5 produzierte Antikörper.

In einer weiteren bevorzugten Ausführungsform ist der 26kDa-Antigen-spezifische Antikörper der von dem bei der Deutschen Sammlung von Mikroorganismen und Zellkulturen (DSMZ) am 23. Juni 1998 nach den Statuten des Budapester Vertrages unter der Hinterlegungsnummer DSM ACC2355 hinterlegten Hybridom HP15m/3E8-D9-D6 produzierte Antikörper.

In einer anderen bevorzugten Ausführungsform ist der β-Urease-spezifische Antikörper der von einem der bei der Deutschen Sammlung von Mikroorganismen und Zellkulturen (DSMZ) am 23. Juni 1998 nach den Statuten des Budapester Vertrages unter den Hinterlegungsnummern DSM ACC2360 oder DSM ACC2362 hinterlegten Hybridomen HP8m/4H5-D4-C9 oder HP9.1m/3C2-F8-E2 produzierte Antikörper. Der in den Beispielen/Figuren beschriebene β-Urease-spezifische Antikörper HP8m/1H5-G2-B4 wird durch einen Tochterklon des hinterlegten Hybridoms HP8m/4H5-D4-C9 produziert. Die beiden durch Mutter- und Tochterklon produzierten Antikörper werden durch identische DNA-Sequenzen kodiert und weisen dieselben Eigenschaften auf.

In einer besonders bevorzugten Ausführungsform weist die schwere Kette des ein Hsp60-Epitop bindenden Antikörpers mindestens eine der folgenden CDRs, vorzugsweise die CDR3 und weiter bevorzugt alle drei folgenden CDRs auf:
- CDR1:: GFSLSRYSVH
- CDR2:: MIWGGGSTDYNSGLKS
- CDR3:: NMGGRYPDYFDY

In einer weiteren besonders bevorzugten Ausführungsform weist die die schwere Kette des ein Hsp60-Epitop bindenden Antikörpers kodierende DNA-Sequenz mindestens eine der folgenden CDRs, vorzugsweise die CDR3 und weiter bevorzugt alle drei folgenden CDRs auf:
- CDR1:: GG GTTCTCATTA TCCAGATATA GTGTACAC
- CDR2:: ATGATATGGG GTGGTGGAAG CACAGACTAT AATTCAGGTC TCAAATCC
- CDR3:: AATATG GGGGGTAGGT ACCCGGACTA CTTTGACTAC

In einer anderen besonders bevorzugten Ausführungsform weist die leichte Kette des ein Hsp60-Epitop bindenden Antikörpers mindestens eine der folgenden CDRs, vorzugsweise die CDR3 und weiter bevorzugt alle drei folgenden CDRs auf:
- CDR1:: RASKSVSTSGYSYIH
- CDR2:: LASNLES
- CDR3:: QHSRELPLT

Weiterhin weist in einer besonders bevorzugten Ausführungsform die die leichte Kette dieses Antikörpers kodierende DNA-Sequenz mindestens eine der folgenden CDRs, vorzugsweise die CDR3 und weiter bevorzugt alle drei folgenden CDRs auf:
- CDR1:: A GGGCCAGCAA GAGTGTCAGT ACATCTGGCT ATAGTTACAT ACAC
- CDR2:: C TTGCATCCAA CCTAGAATCT
- CDR3:: CAGC ACAGTAGGGA GCTTCCGCTC ACG.

In einer weiteren besonders bevorzugten Ausführungsform des erfindungsgemäßen Verfahrens weist die schwere Kette des ein 26kDa-Protein bindenden Antikörpers mindestens eine der folgenden CDRs, vorzugsweise die CDR3 und weiter bevorzugt alle drei folgenden CDRs auf:
- CDR1:: GFTFNSYAMY
- CDR2:: RIRSKSDNYATYYANSVKD
- CDR3:: DHDKFPFYYALDY

In einer anderen besonders bevorzugten Ausführungsform weist die die schwere Kette des ein Epitop des 26kDa-Proteins bindenden Antikörpers kodierende DNA-Sequenz mindestens eine der folgenden CDRs, vorzugsweise die CDR3 und weiter bevorzugt alle drei folgenden CDRs auf:
- CDR1:: GG TTTCACCTTC AATTCCTATG CCATGTAC
- CDR2:: CGCATAAGAA GTAAAAGTGA TAATTATGCA ACATATTATG CCAATTCAGT GAAAGAC
- CDR3:: GATCATG ATAAGTTTCC TTTTTACTAT GCTCTGGACT AC

In einer anderen besonders bevorzugten Ausführungsform des erfindungsgemäßen Verfahrens wird ein Antikörper, Fragment oder Derivat davon eingesetzt, dessen leichte Kette des ein 26kDa-Protein bindenden Antikörpers mindestens eine der folgenden CDRs, vorzugsweise die CDR3 und weiter bevorzugt alle drei folgenden CDRs aufweist:
- CDR1:: TASSSVSSSYLH
- CDR2:: STSNLAS
- CDR3:: HQYHRSPPT

Weiterhin weist die die leichte Kette dieses Antikörpers kodierende DNA-Sequenz in einer anderen besonders bevorzugten Ausführungsform mindestens eine der folgenden CDRs, vorzugsweise die CDR3 und weiter bevorzugt alle drei folgenden CDRs auf:
- CDR1:: A CTGCCAGCTC AAGTGTGAGT TCCAGTTACT TGCAC
- CDR2:: AGCACTTCCA ACCTGGCTTC T
- CDR3:: CAC CAGTATCATC GTTCCCCACC GACG

In einer anderen besonders bevorzugten Ausführungsform des erfindungsgemäßen Verfahrens weist die schwere Kette des ein Epitop der β-Urease bindenden Antikörpers mindestens eine der folgenden CDRs, vorzugsweise die CDR3 und weiter bevorzugt alle drei folgenden CDRs auf:
- CDR1:: GFTFSSHFMS
- CDR2:: SISSGGDSFYPDSLKG
- CDR3:: DYSWYALDY
oder:
- CDR1:: GYAFSTSWMN
- CDR2:: RIYPGDGDTNYNGKFKG
- CDR3:: EDAYYSNPYSLDY

In einer weiterhin besonders bevorzugten Ausführungsform weist die die schwere Kette des ein Epitop der β-Urease bindenden Antikörpers kodierende DNA-Sequenz mindestens eine der folgenden CDRs, vorzugsweise die CDR3 und weiter bevorzugt alle drei folgenden CDRs auf:
- CDR1:: GG CTACGCATTC AGTACCTCCT GGATGAAC
- CDR2:: CGGATTTATC CTGGAGATGG AGATACTAAC TACAATGGGA AGTTCAAGGG C
- CDR3:: GAG GATGCCTATT ATAGTAACCC CTATAGTTTG GACTAC
oder:
- CDR1:: GG ATTCACTTTC AGTAGCCATT TCATGTCT
- CDR2:: TCCATTAGTA GTGGTGGTGA CAGTTTCTAT CCAGACAGTC TGAAGGGC
- CDR3:: GACTAC TCTTGGTATG CTTTGGACTA C

In einer anderen besonders bevorzugten Ausführungsform des erfindungsgemäßen Verfahrens weist die leichte Kette des ein Epitop der β-Urease bindenden Antikörpers mindestens eine der folgenden CDRs, vorzugsweise die CDR3 und weiter bevorzugt alle drei folgenden CDRs auf:
- CDR1:: RASQSIGTRIH
- CDR2:: YGSESIS
- CDR3:: QQSNTWPLT
oder:
- CDR1:: HASQNINVWLS
- CDR2:: KASNLHT
- CDR3:: QQGRSYPLT

Des weiteren weist die die leichte Kette dieses Antikörpers kodierende DNA-Sequenz bevorzugt folgende CDRs auf:
- CDR1:: A GGGCCAGTCA GAGCATTGGC ACAAGAATAC AC
- CDR2:: TAT GGTTCTGAGT CTATCTCT
- CDR3:: CAACAA AGTAATACCT GGCCGCTCAC G
oder:
- CDR1:: C ATGCCAGTCA GAACATTAAT GTTTGGTTAA GC
- CDR2:: AAG GCTTCCAACT TGCACACA
- CDR3:: CAACAG GGTCGAAGTT ATCCTCTCAC G

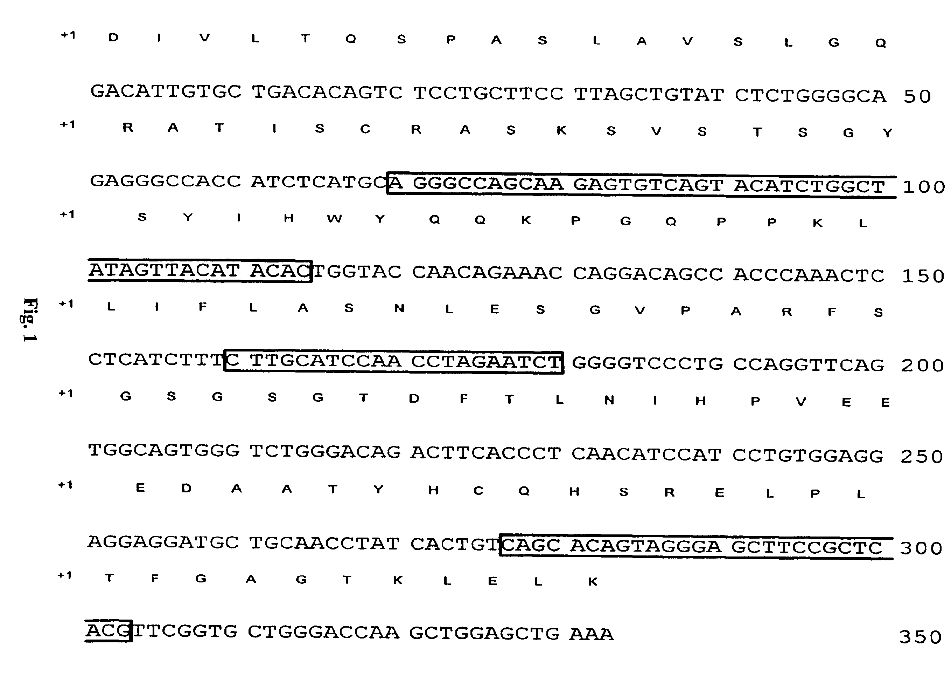

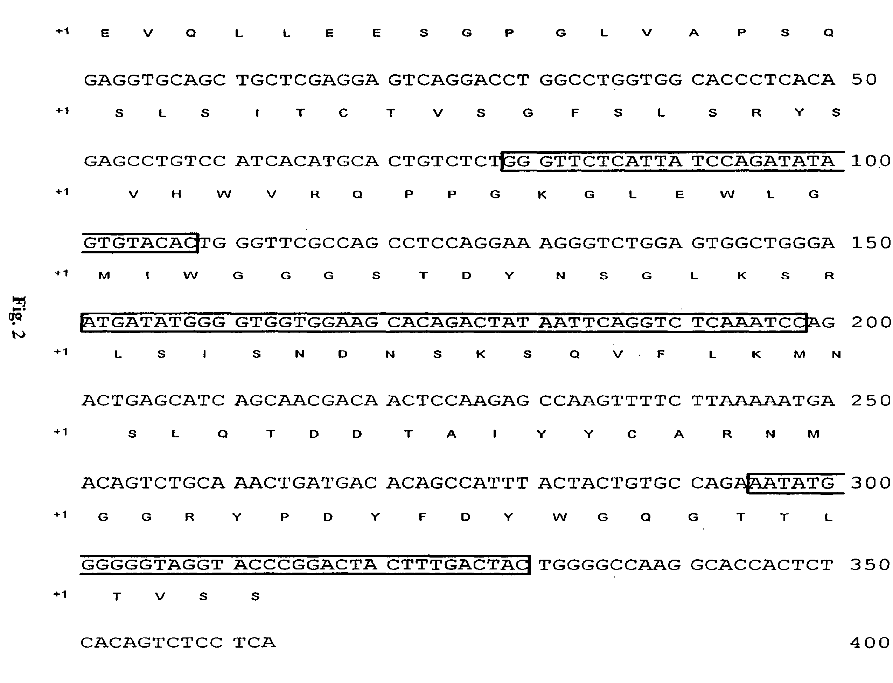

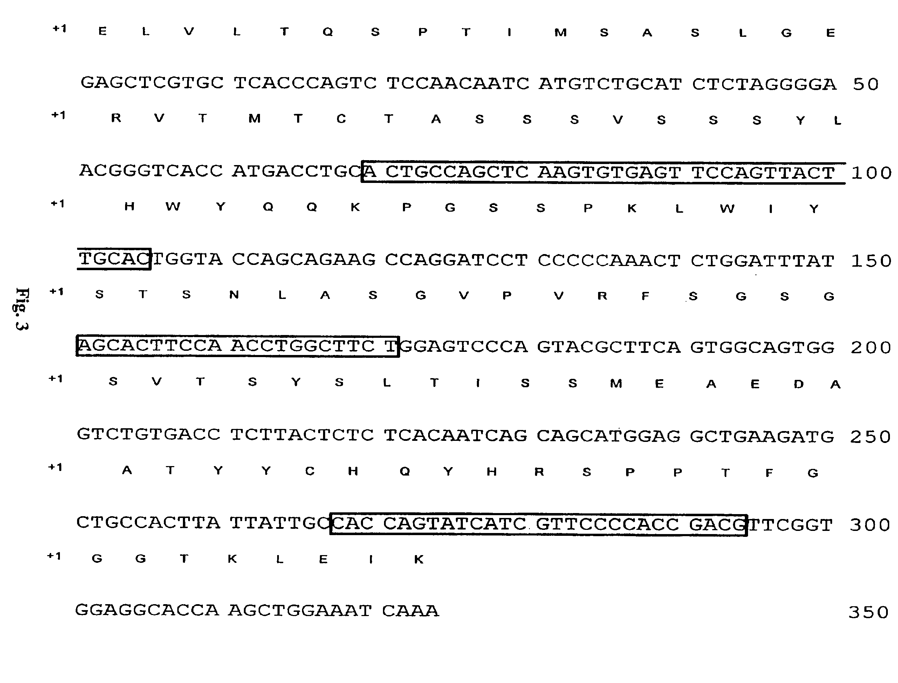

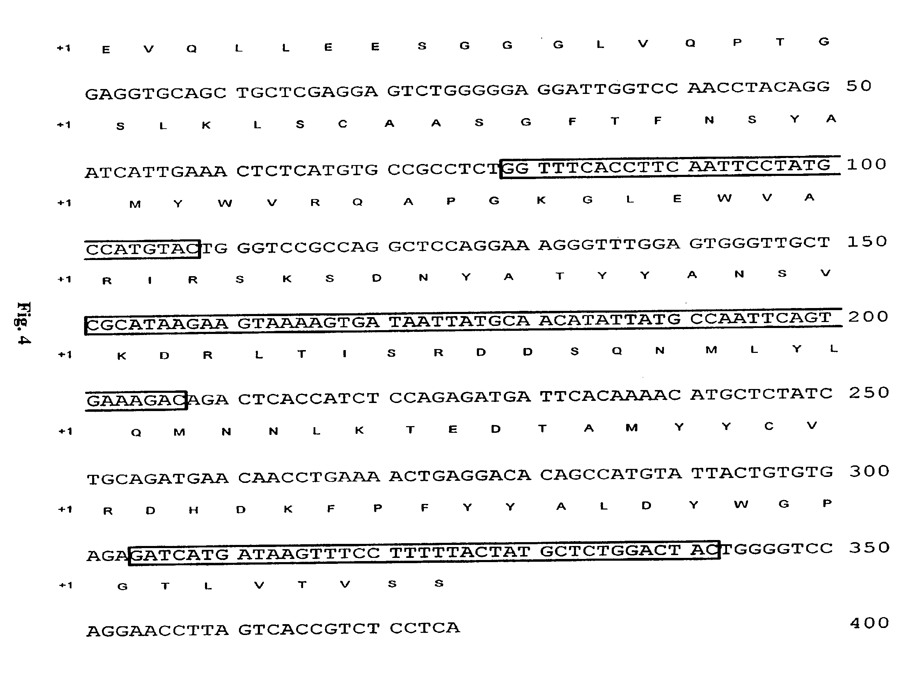

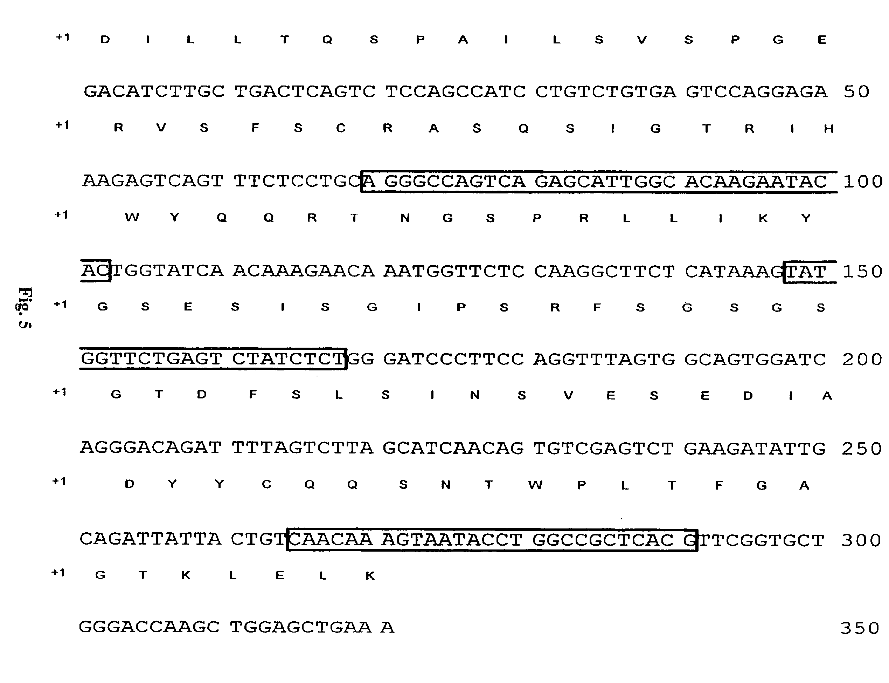

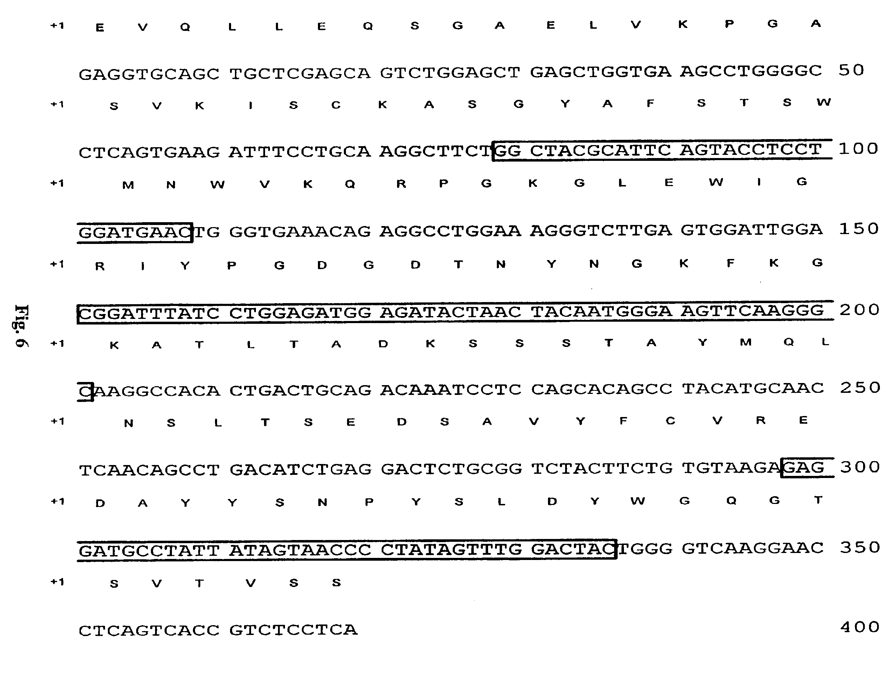

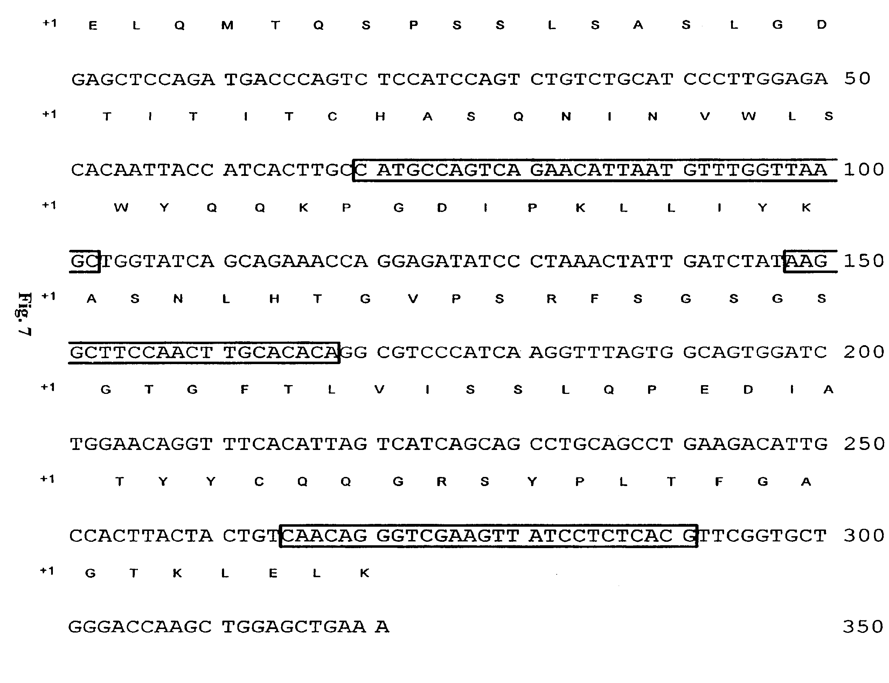

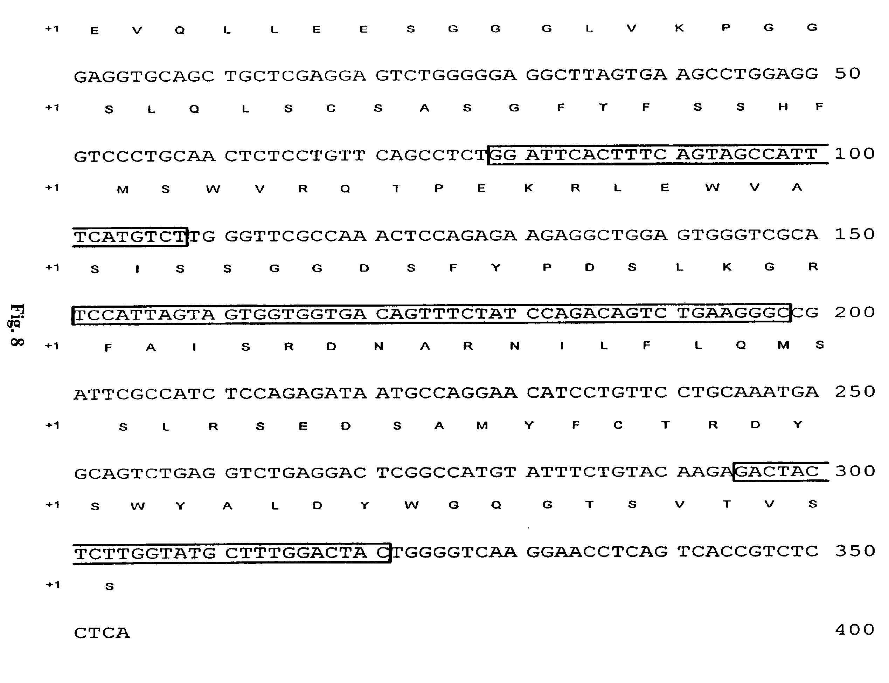

Besonders bevorzugt ist ferner, daß die schweren und leichten Ketten, die die vorstehend angegebenen CDRs aufweisen, gemeinsam in einem Antikörper, Fragment oder Derivat davon auftreten, der/das Hsp60, das 26kDa-Protein oder die β-Urease, oder ein Fragment davon, vorzugsweise aus *H. pylori* spezifisch bindet. Die Erfindung umfaßt jedoch auch Ausführungsformen, in denen diese schweren oder leichten Ketten mit anderen leichten bzw. schweren Ketten kombiniert werden, wobei die Bindungseigenschaften im wesentlichen beibehalten oder verbessert werden können. Entsprechende Verfahren sind im Stand der Technik bekannt. Besonders bevorzugte Antikörper weisen in den variablen Regionen der leichten und schweren Ketten die in den Figuren 1 und 2, den Figuren 3 und 4, den Figuren 5 und 6 oder den Figuren 7 und 8 dargestellten Aminosäuresequenzen auf bzw. werden die Regionen von den dort dargestellten DNA-Sequenzen kodiert.

In einer bevorzugten Ausführungsform werden mit der Stuhlprobe vor der Inkubation mit den Antikörpern folgende Schritte durchgeführt: Die Stuhlprobe wird 1:3 bis 1:25, vorzugsweise etwa 1:10 in einem Resuspendierungspuffer resuspendiert und daraufhin auf einem Vortexmixer gemischt. Ein Beispiel für einen Resuspendierungspuffer ist, 150 mM PBS, 0,1% SDS.

In einer weiteren bevorzugten Ausführungsform erfolgt der Nachweis der Bildung des mindestens einen Antigen-Antikörperkomplexes/Antigen-Aptamerkomplexes in Schritt (b) mittels eines immunologischen Verfahrens.

In einer anderen bevorzugten Ausführungsform erfolgt der Nachweis der Bildung des mindestens einen Antigen-Antikörperkomplexes/Antigen-Aptamerkomplexes in Schritt (b) mittels ELISA, RIA, Western Blot oder eines immunchromatographischen Verfahren.

Derartige Verfahren sind an sich im Stand der Technik bekannt; vgl. Harlow und Lane, a.a.O.

In einer besonders bevorzugten Ausführungsform des erfindungsgemäßen Verfahrens wird im immunologischen Verfahren, insbesondere im RIA oder im ELISA derselbe Antikörper oder dessen Fragment oder Derivat oder das Aptamer zur Bindung an die Festphase wie auch zum Nachweis des Epitops eingesetzt. Während der Fängerantikörper/das Fängeraptamer in unmodifizierter Form an die Festphase, beispielsweise eine Mikrotiterplatte, gebunden werden kann, ist der zur Detektion eingesetzte Antikörper, das Fragment oder Derivat davon bzw. das Aptamer gegebenenfalls mit einer Markierung versehen. Andererseits kann dieser Antikörper, das Fragment oder Derivat davon oder dieses Aptamer ebenfalls nicht markiert sein und damit das Epitop des Mikroorganismus, vorzugsweise das bakterielle Epitop auch über einen dritten markierten Antikörper, das Fragment oder Derivat davon ein drittes markiertes Aptamer nachgewiesen werden, wobei dieser Antikörper, das Fragment oder Derivat davon oder dieses Aptamer ein speziesspezifischer oder Ig-klassenspezifischer Antikörper oder ein entsprechendes Aptamer sein kann. Markierungen von Antikörpern, beispielsweise mit radioaktiven oder fluoreszierenden Markern sind im Stand der Technik bekannt; vgl. Harlow und Lane a.a.O. Entsprechendes gilt für Aptamere. Die vorstehend beschriebenen Ausführungsform ist besonders günstig zum Nachweis der Urease, vorzugsweise β-Urease, die ggf. auch nach der Darmpassage noch als Dimer, ggf. in multimerer Ausführung vorliegt. Selbstverständlich können auch in dieser Ausführungsform Kombinationen von Antikörpern, Fragmenten, Derivaten und Aptameren eingesetzt werden, z.B. Kombinationen von Antikörpern etc., die an unterschiedliche Epitope desselben Antigens binden.

In einer anderen bevorzugten Ausführungsform des erfindungsgemäßen Verfahrens ist der monoklonale Antikörper ein Maus-Antikörper.

Des weiteren sind in einer bevorzugten Ausführungsform die Antikörper, Fragmente oder Derivate davon oder die Aptamere an einen Träger fixiert.
Die Fixierung der Antikörper, Fragmente oder Derivate davon oder der Aptamere an einen Träger ist besonders vorteilhaft für die Durchführung von Routinechecks. Die Kombination Antikörper-Träger/Aptamer-Träger läßt sich ferner gut als Testbesteck oder in Kitform verpacken.

In einer besonders bevorzugten Ausführungsform ist das Trägermaterial ein poröses Trägermaterial.

In einer weiteren besonders bevorzugten Ausführungsform ist das Trägermaterial ein Teststreifen.

Zusätzlich besteht in einer bevorzugten Ausführungsform das Trägermaterial aus Zellulose oder einem Zellulosederivat.

Der Säuger, dessen Stuhl mit dem erfindungsgemäßen Verfahren untersucht werden kann, kann ein Tier, beispielsweise ein Haustier wie eine Katze oder ein Hund, ein Nutztier, z.B. ein Schwein oder ein sonstiges Tier wie eine Maus, ein Tiger oder ein Frettchen sein.

In einer bevorzugten Ausführungsform ist der Säuger ein Mensch.

Darüber hinaus betrifft die Erfindung einen monoklonaler Antikörper, ein Fragment oder Derivat davon, der/das eine V-Region aufweist, die eine Kombination der vorstehend dargestellten CDRs aufweist oder der von einem der vorstehend dargestellten Hybridomen produziert wird.

Bevorzugt ist dabei ein monoklonaler Antikörper, Fragment oder Derivat davon, der/das mindestens eine der in den Figuren 1 bis 8 dargestellten V-Regionen aufweist. Vorzugsweise weist dieser Antikörper zwei der in den Figuren 1 und 2, 3 und 4, 5 und 6 oder den Figuren 7 und 8 dargestellten V-Regionen auf. Auch ist bevorzugt, daß diese V-Regionen von den in den Figuren 1 bis 8 dargestellten DNA-Sequenzen kodiert werden.

In einer besonders bevorzugten Ausführungsform der Erfindung ist der monoklonale Antikörper, das Fragment oder Derivat davon ein Maus-Antikörper oder ein Fragment oder Derivat davon oder ein chimärer, vorzugsweise ein humanisierter Antikörper oder ein Fragment oder Derivat davon. Das Derivat kann auch ein Fusionsprotein sein. Weiter bevorzugt ist, daß der Antikörper markiert ist, beispielsweise mit einem Kolloid, mit einer radioaktiven, fluoreszierenden, phosphoreszierenden oder chemiluminiszierenden Markierung.
Die Herstellung von chimärisierten humanisierten und humanen Antikörpern und der anderen Derivate ist im Stand der Technik wohlbekannt (z.B. Vaughan et al., 1998; Orlandi et al., 1989, Harlow und Lane, a.a.O.).

Die Erfindung betrifft auch ein Aptamer, das dasselbe Epitop wie der monoklonale Antikörper, das Fragment oder Derivat davon spezifisch bindet. Die Herstellung derartiger Aptamere kann mit im Stand der Technik bekannten Verfahren erfolgen.

Darüber hinaus betrifft die Erfindung weitere Antikörper, Derivate oder Fragmente davon, die das erfindungsgemäße Epitop spezifisch binden. Diese Antikörper können beispielsweise monoklonale Antikörper sein, die unter Verwendung des Epitops als Hapten/Bestandteil eines Antigens nach üblichen Verfahren hergestellt werden können.

Die vorliegende Erfindung betrifft darüber hinaus eine diagnostische Zusammensetzung enthaltend mindestens zwei monoklonale Antikörper, Fragmente oder Derivate davon oder Aptamere wie oben stehend definiert, gegebenenfalls fixiert an ein Trägermaterial.

Des weiteren betrifft die vorliegende Erfindung eine Testvorrichtung zum Nachweis mindestens eines wie oben stehend definierten Epitops, umfassend (a) mindestens zwei monoklonale Antikörper, Fragmente oder Derivate davon oder Aptamere wie obenstehend definiert, fixiert an ein Trägermaterial; (b) eine Vorrichtung zur Aufbereitung und Analyse von Stuhlproben und gegebenfalls (c) ein Gemisch von mindestens zwei monoklonalen Antikörpern, Fragmenten oder Derivaten davon oder Aptameren.

Die Erfindung hat ferner zum Gegenstand eine Testvorrichtung enthaltend (a) mindestens zwei monoklonale Antikörper, Fragmente oder Derivate davon oder Aptamere wie obenstehend definiert, wobei die Antikörper, Fragmente oder Derivate davon oder Aptamere konjugiert mit kolloidalem Gold, Latexpartikeln oder anderen farbgebenden Partikeln sind, deren Größe typischerweise im Bereich zwischen 5nm und 100nm, vorzugsweise zwischen 20nm und 60nm liegt; (b) eine Vorrichtung zur Aufbereitung und Analyse von Stuhlproben und gegebenfalls (c) ein Gemisch von mindestens zwei monoklonalen Antikörpern, Fragmenten oder Derivaten davon oder Aptameren.

Darüber hinaus betrifft die vorliegende Erfindung einen Kit enthaltend (a) mindestens zwei monoklonale Antikörper, Fragmente oder Derivate davon oder Aptamere wie obenstehend definiert, gegebenenfalls fixiert an ein Trägermaterial; gegebenenfalls (b) eine Vorrichtung zur Aufbereitung und Analyse von Stuhlproben und gegebenfalls (c) ein Gemisch von mindestens zwei monoklonalen Antikörpern, Fragmenten oder Derivaten davon oder Aptameren.

Schließlich betrifft die Erfindung eine Packung enthaltend die erfindungsgemäße diagnostische Zusammensetzung, die erfindungsgemäße Testvorrichtung oder den erfindungsgemäßen Kit.

Die Bestandteile der erfindungsgemäßen diagnostischen Zusammensetzung, der erfindungsgemäßen Testvorrichtung und/oder des erfindungsgemäßen Kits können in Behältern, wie beispielsweise Fläschchen oder Röhrchen, gegebenenfalls in Puffern und/oder Lösungen verpackt sein. Unter Umständen können eine oder mehrere der Bestandteile in ein- und demselben Behälter verpackt sein.

Die Figuren zeigen:
Fig. 1: DNA-Sequenz, die für eine leichte Kette eines für HSP60 spezifischen monoklonalen Antikörpers (DMS ACC2356) kodiert. Die DMSZ-Hinterlegungsnummer bezieht sich hier wie auch sonst in der Anmeldung auf das den monoklonalen Antikörper produzierende Hybridom. Die kodierte Aminosäuresequenz ist ebenfalls dargestellt.
Fig. 2: DNA-Sequenz, die für eine schwere Kette eines für HSP60 spezifischen monoklonalen Antikörpers (DMS ACC2356) kodiert. Die kodierte Aminosäuresequenz ist ebenfalls dargestellt.
Fig. 3: DNA-Sequenz, die für eine leichte Kette eines für 26kDa-Protein spezifischen monoklonalen Antikörpers (DMS ACC2355) kodiert. Die kodierte Aminosäuresequenz ist ebenfalls dargestellt.
Fig. 4: DNA-Sequenz, die für eine schwere Kette eines für 26kDa-Protein spezifischen monoklonalen Antikörpers (DMS ACC2355) kodiert. Die kodierte Aminosäuresequenz ist ebenfalls dargestellt.
Fig. 5: DNA-Sequenz, die für eine leichte Kette eines ersten für Urease spezifischen monoklonalen Antikörpers (DMS ACC2360) kodiert. Die kodierte Aminosäuresequenz ist ebenfalls dargestellt.
Fig. 6: DNA-Sequenz, die für eine schwere Kette eines ersten für Urease spezifischen monoklonalen Antikörpers (DMS ACC2360) kodiert. Die kodierte Aminosäuresequenz ist ebenfalls dargestellt.
Fig. 7: DNA-Sequenz, die für eine leichte Kette eines zweiten für Urease spezifischen monoklonalen Antikörpers (DMS ACC2362) kodiert. Die kodierte Aminosäuresequenz ist ebenfalls dargestellt.
Fig. 8: DNA-Sequenz, die für eine schweren Kette eines zweiten für Urease spezifischen monoklonalen DMS ACC2362) kodiert. Die kodierte Aminosäuresequenz ist ebenfalls dargestellt.

Die Beispiele erläutern die Erfindung.

### Beispiel 1: Isolierung von H. pylori-Antigenen

### Kultivierung von H. pylori

*H. pylori* (Stamm NCTC 11637) wurde in Petri-Schalen auf Wilkins-chalkern Agar unter Zusatz von 10% Pferdeblut sowie Amphotericin B, Vancomycin und Cefsoludin (Sigma Chemicals) ausgestrichen und 3-4 Tage in mikroaerophiler Atmosphäre (Anaerocult GasPAk, Merck) bei 37ºC inkubiert. Der Inhalt von 2 Schalen wurde in 350ml BHIB-Medium unter Zusatz der Antibiotika wie oben in einer 11 Flasche (Schott) suspendiert, das Medium für 10min mit einem Gasgemisch aus 10% CO₂, 5% O₂, 85% N₂ begast und die Flasche verschlossen. Die Kultur wurde für 2 Tage bei 37 ºC auf einem Rundschüttler geschüttelt, nach 24h wurde der Verschluß der Flasche geöffnet. Der Inhalt der Flasche wurde anschließend steril in eine 101 Flasche gegeben, mit 4,7l BHIB-Medium aufgefüllt, das Medium für 10min mit einem Gasgemisch aus 10% CO₂, 5% O₂, 85% N₂ begast und die Flasche verschlossen. Wiederum wurde für 2 Tage bei 37ºC auf einem Rundschüttler geschüttelt, nach 24h wurde der Verschluß der Flasche geöffnet. Das gesamte Volumen wird daraufhin bei 11000g für 10 min zentrifugiert, Überstand dekantiert und das Bakterienpellet gewogen. Zur Lagerung wurde der Pellet in einer physiologischen Kochsalzlösung unter Zusatz von 15% Glycerin im Verhältnis 2:1 (Gew.:Vol.) resuspendiert und bei -80ºC eingefroren. Um die Identität der kultivierten Bakterien zu überprüfen wurde eine visuelle Inspektion der Bakterien sowie Tests auf Urease-, Oxidase und Katalase-Aktivität durchgeführt. Die Freiheit von Kontamination wurde durch Kultur von etwas Suspension, entnommen vor dem Einfrieren, bei 37ºC an Luft für 48h belegt.

### Beispiel 2: Allgemeine Vorgehensweise zur Auswahl geeigneter Antigene zur Detektion auf Mikroorganismen

Nach Aufzucht wird ein Lysat des Mikroorganismus hergestellt. Die Auftrennung des Lysats erfolgt mittels Gelfiltration. Anschließend werden diejenigen Proteine isoliert, die in dem Lysat in großer Menge vorliegen. Diese Proteine werden mittels Proteinsequenzierung und den Vergleich mit Sequenzen aus geeigneten Datenbanken identifiziert. Hierdurch können charakteristische bzw. spezifische Proteine für den jeweiligen Mikroorganismus ermittelt werden. Für diese ausgewählten Antigene wird dann ein geeignetes Reinigungsschema entwickelt.

### Beispiel 3: Präparation von H. pylori Urease, Alkylhydroperoxid-Reduktase und HSP60

Die Präparation der drei *H. pylori*-Antigene Urease, Alkylhydroperoxid-Reduktase und HSP60 entspricht einer Modifikation einer bekannten Methode (Eschweiler et al., 1993). Gefrorenes Bakterienpellet wurde im Verhältnis 1:2 (Gew.:Vol.) mit 10% Butanol versetzt, auf einem Über-Kopf-Mischer bis zum vollständigen Auftauen und ca. 15min weitergeschüttelt. Nach Sedimentation für 20min bei 20.000g, 4°C, wurde der Überstand dekantiert und das Pellet erneut für 30min mit 10% Butanol wie oben extrahiert. Nach erneuter Sedimentation analog wie oben wurden die Überstände vereinigt und das Pellet zur weiteren Verwendung eingefroren.

### Isolierung von Urease und Alkylhydroperoxid-Reduktase (als Oligomere)

Die vereinigten klaren Überstände wurden durch 0,8µm Filter filtriert und sofort auf Source Q 16/10-Säule (mit 20mM HEPES, pH7.0, equilibriert) überführt. Mit 20mM HEPES, pH7.0, 2M NaCl eluierte Fraktionen wurden unmittelbar mittels eines Schnelltests auf Urease-Aktivität überprüft (4ml 1 M Harnstoff, 1ml 0,2M HEPES, pH7,0 sowie 80µl einer Lösung von 5g/l Phenolrot wurden vermischt und mit aqua dest. auf 20ml aufgefüllt. 100µl dieser Lösung wurden anschließend mit der gleichen Menge einer Fraktion in ein Reagenzglas gegeben. Der Farbumschlag von gelb nach rot zeigte die Anwesenheit von Urease an). Urease-positive Fraktionen wurden auf eine mit Sephacryl S200 50/100 (Pharmacia) gefüllte Chromatographiesäule gegeben und aufgetrennt (Puffer: 50mM Tris, 100mM NaCl, 0,05% NaN₃, pH 7,3). Die Proteinkonzentration im Auslauf wurde durch Messung der optischen Dichte bei 280nm überwacht. Die Fraktionen des ersten Protein-Peaks enthielten oligomere Urease (Molekulargewicht ca. 550-600 kDa), gefolgt von einem zweiten Peak, das der Alkylhydroperoxid-Reduktase (ebenfalls als Oligomer) zuzurechnen war. Überschneidende Fraktionen wurden verworfen, reine Fraktionen (Überprüfung durch Elektrophorese auf Polyacrylamid-Gel) vereinigt und im Vakuum (Speed-Vac) oder durch Ultrafiltration eingeengt. Alkylhydroperoxid-Reduktase-Fraktionen, die noch größere Mengen Urease enthielten, wurden nachgereinigt mit Hilfe einer β-Urease Affinitätssäule (Antikörper Hp8m/1H5-G2B4, Connex, auf CNBr-aktivierter Sepharose 4B-Matrix, Amersham Pharmacia, Kopplung nach Angaben des Herstellers).

### Isolierung von HSP60 (als Oligomer)

Das HSP60 Antigen konnte nicht in ausreichender Quantität mit der oben beschriebene Butanol-Extraktionsmethode gewonnen werden. Deshalb wurden die Bakterien mit Ultraschall aufgeschlossen. Frisches Bakterienpellet oder das nach der Extraktion mit 10% Butanol erhaltene Pellet wurde aufgetaut, im Verhältnis 1:10 in 150mM PBS, pH 7,5 resuspendiert und in einem Falcon-Röhrchen auf Eiswasser in einem Sonikator (Sonifire) auf Stufe 25-30% (Stufe 7) Ultraschall-behandelt (4x90s mit je 90s Pause). Nach Zentrifugation bei 20.000g für 40min wurde der Überstand auf eine mit Sephacryl S200 50/100 (Pharmacia) gefüllte Chromatographiesäule aufgetragen und aufgetrennt (Puffer: 50mM Tris, 100mM NaCl, 0,05% NaN₃, pH7,3). HSP60 koeluierte mit Urease, daher wurden Urease-positive (siehe oben) Fraktionen vereinigt und auf einer β-Urease Affinitätssäule (siehe oben) nachgereinigt.

### Beispiel 4: Herstellung polyklonaler und monoklonaler Antikörper (pAK; mAK) gegen H. pylori-spezifische Antigene

Polyklonale Antiseren wurden von pab Productions (Hebertshausen) nach Standardverfahren gegen wie oben gereinigte Antigene hergestellt. Die Herstellung monoklonaler Antikörper erfolgt nach Methoden, die dem Fachmann bekannt sind (Harlow & Lane, 1988; Peters & Baumgarten, 1990).

### Immunisierung

Als Immunogen eingesetzt wurden die gereinigten Proteine Urease, HSP60 und Alkylhydroperoxid-Reduktase sowie rekombinante α- und β-Urease (Austral Biologics). Pro Immunogen wurden 4-6 Mäuse (BALB/c x C57 Black, F1-Generation, 8-12 Wochen alt) immunisiert (Priming) und in Abständen von ca. vier Wochen 3-4x geboostet. Pro Injektion wurden 200-300µl Antigenlösungen (25-50µg Antigen/Tier) im Verhältnis 1:1 mit kompletten (Priming) bzw. inkompletten (Boost) Freundschen Adjuvans (Difco) emulgiert und 100µl/Maus intraperitoneal injiziert. Vor der Fusion wurde den Mäusen retroorbital Blut entommen und aus dem daraus gewonnenen Antiserum der Antikörpertiter mittels Enzym-linked Immunosorbent Assay (ELISA, siehe unten) bestimmt.

### Fusion und Fusionsscreening

2-3 Tage nach dem letzten Boost wurden die Milz-Zellen der immunisierten Mäuse mit den Myelomzellen P3x63Ag8.653 (ATCC CRL-1580; Kearney et al., 1979) im Verhältnis 5:1 mit Polyethylenglykol 4000 fusioniert. Die fusionierten Zellen wurden in Klonierungsmedium (RPMI 1640 Medium+ 20% FCS + 200U IL-6/ml) mit Hypoxanthin-Aminopterin-Thymidin-Supplement (100x Konzentrat, Sigma) suspendiert und mit einer Zelldichte von 2-6x10⁴ Zellen/Napf auf 96-Napf-Mikrotiterplatten in Kultur genommen (37°C, 6% CO₂ und 95% relativer Luftfeuchtigkeit).

Nach ca. 10 Tagen wurde der Kulturüberstand im ELISA (siehe unten) auf das Vorhandensein von Antikörpern der gewünschten Spezifität gescreent.

### Etablierung und Kultivierung der Hybridome

Klone, die antigenspezifische Antikörper produzieren, wurden zweimal nach dem Prinzip der Grenzverdünnung (Limiting Dilution) (Coller & Coller, 1983) in Klonierungsmedium mit Hypoxanthin-Thymidin-Supplement (100x Konzentrat, Sigma) rekloniert. Der monoklonale Endklon wurde anschließend an die Flachflaschenkultur in RPMI 1640 Medium mit 10%FCS adaptiert und für die Kryokonservierung von 5-10 Aliquots mit je 2-5x10⁶ Zellen und die Produktion von antikörperhaltigen Kulturüberständen expandiert.

### ELISA

Für die Titerbestimmung und den Test der Kulturüberstände auf antigenspezifische Antikörper wurde der direkte ELISA gewählt. Die Beschichtung der ELISA-Platten (MaxiSorb; Nunc) erfolgte ü.N. bei 5°C mit einer Antigensuspension (2-6 µg Antigen/ml Carbonatpuffer, 0,1 M, pH9,5). Zur Blockade der noch freien Bindungsstellen wurden 200µl PBS mit 2% Magermilchpulver (Gew.:Vol.) pro Napf pipettiert und 1 h bei Raumtemperatur inkubiert. Die Inkubation von 100µl Kulturüberstand erfolgte 1-2h bei Raumtemperatur. Die Detektion der gebundenen Antikörper erfolgt durch Zugabe eines mit Meerrettich-Peroxidase (POD) konjugierten Sekundärantikörpers (Kaninchen-anti-Maus IgG-POD, DAKO). Die POD setzt dann im nächsten Schritt das farblose Substrat Tetramethylbenzidin (TMB, Sigma) in ein blaues Produkt um. Nach 5 bis 10 Minuten, oder sobald auch die Negativkontrolle eine leichte Blaufärbung zeigte, wurde die Reaktion durch Zugabe von 1 N Schwefelsäure (100 µl/Napf) gestoppt. Die Stärke der Farbreaktion wurde im ELISA-Reader (MWG Spektral) gemessen. Die Messung erfolgt bei 455 nm gegen die Referenzwellenlänge 620 nm. Zwischen den einzelnen Arbeitsschritten wurde die ELISA-Platte 2-3x mit 250µl PBS mit 0,025% Tween 20 (Vol.:Vol.) gewaschen.

### Ergebnisse

Insgesamt wurden 24 mAK gegen β-Urease, 6 mAK gegen α-Urease, 8 mAK gegen 26kDa-Protein sowie 10 mAK gegen HSP60 generiert. Aus diesem Antikörper-Repertoire wurden diejenigen mAK mit den niedrigsten Nachweisgrenzen für die jeweiligen Antigene ausgewählt.

### Beispiel 5: Reinigung von mAK aus Hybridomakulturüberständen und Herstellung von Konjugaten

Die Reinigung von mAK aus Hybridomakulturüberständen erfolgt mittels der Protein-G Affinitätschromatographie (modifiziert nach: Pharmacia Biotech, 1994).

Die Kulturüberstände wurden filtriert (0,8µm) und direkt über die Protein G Matrix geleitet. Gewaschen wurde mit Tris/HCl bis das Signal am Detektor zum Hintergrund zurückgekehrt war. Eluiert wurde mit 0,1 M Glycin/HCl, pH 3,0, die Detektion der Proteinkonzentration erfolgte im Eluat über optische Dichte bei 280nm. Alle Fraktionen im Bereich des einzigen Elutionssignals können verwendet werden.

Eine mögliche Verunreinigung mit bovinen IgG aus dem Serumzusatz ist als unkritisch einzustufen, da die gefundenen Nachweisantikörper nicht mit Rinder-Ig kreuzreagieren.

Die Kopplung monoklonaler Antikörper an Biotin und POD erfolgte nach Literaturmethoden (Harlow & Lane, 1988).

### Beispiel 6: Charakterisierung der monoklonalen Antikörper

### Isotypisierung

Die murinen mAK wurden mit dem Isotyping Kit IsoStrip von Boehringer Mannheim (Mannheim) isotypisiert.

### Immunblot

Für die Immunblotanalyse wurden pro Gel 30-50µg gereinigtes Antigen bzw. 300µg *H. pylori*-Lysat in reduzierendem Probenpuffer (Laemmli, 1970) gekocht und auf ein 12%iges präparatives SDS-Poplyacrylamid-Minigel (8,6x7,7x0,1cm, Biometra) aufgetragen. Die elektrophoretische Auftrennung erfolgte bei 25-30 mA/Gel.

Die im Gel aufgetrennten Proteine (Antigene) wurden dann im Semidry-Blot-Verfahren auf einer Nitrozellulose-Membran immobilisiert.

Die Membran wurde 30min bei Raumtemperatur mit 2% Magermilchpulver in PBS geblockt und dreimal 5min mit TBS/Tween 20 (0,2%) gewaschen. Für den folgenden Inkubationsschritt wurde die Membran in die Accutran Cross-Blot-Screening-Einheit (Schleicher und Schüll) eingespannt, unter Verwendung einer Gitterplatte mit 34 Querkanälen. In jeden der entstandenen Querkanäle wurden 250µl TBS/Tween 20 vorgelegt und je 250µl der zu testenden Hybridomakulturüberstände zugegeben. Die Inkubation erfolgte 2h bei Raumtemperatur unter Agitation.

Nach dreimaligem Waschen (s.o.) wurde die Membran 1h mit dem POD-konjugierten Sekundärantikörper (Kaninchen-anti-Maus IgG-POD, DAKO) inkubiert.

Die Membran wurde dreimal gewaschen und der Immunkomplex durch Zugabe der 3,3-Diaminobenzidine-Substratlösung (DAB, Sigma) visualisiert.

### Charakterisierung von Antikörper-Antigen-Wechselwirkungen mittels Epitop-Kartierung

Um die Antikörperbindungsstelle an einem Protein identifizieren zu können, wurden von den *H. pylori*-Proteinen α- und β-Urease, HSP60 und 26kDa-Protein überlappende Peptidsequenzen (jeweils zwei Aminosäuren gegeneinander verschoben) mit 12 Aminosäureresten an einer Festphase synthetisiert und kovalent an eine Celluloseazetatmembran gekoppelt (Jerini GmbH, Berlin). Diese Membranen wurden mit Hybridomakulturüberstand inkubiert und die gebundenen Antikörper anschließend im Semidry-Blot Verfahren auf Nitrocellulosemembranen geblottet. Die Membran wurde geblockt und die immobilisierten Antikörper wie unter 5.2 beschrieben mit einem POD-markierten Sekundärantikörper (Kaninchen-anti Maus IgG-POD, DAKO) detektiert.

### Bestimmung der Nachweisgrenze für H. pylori-Antigene im ELISA

Zwischen den einzelnen Arbeitsschritten wurde die ELISA-Platte 2-3x mit 300µl PBS mit 0,025% Tween (Waschpuffer) (Vol.:Vol.) gewaschen. Die Beschichtung der ELISA-Platten (MaxiSorb; Nunc) erfolgte 1 h bei 37ºC mit 100 µl einer Lösung eines polyklonalen Kaninchen-anti-*H. pylori*-Antigen Antikörpers (pAK; ca. 10µg Antikörper/ml Carbonatpuffer, 0,1M, pH9,5). Zur Blockade der noch freien Bindungsstellen wurden 200µl 150mM PBS mit 2% Magermilchpulver (Gew.:Vol.) pro Napf pipettiert und 30min bei Raumtemperatur inkubiert. 50ng/ml gereinigte *H. pylori*-Antigene gelöst in 150mM PBS unter Zusatz von 0,1% Magermilchpulver wurden in 1:2 Schritten verdünnt. Puffer diente als Negativ-Kontrolle. Anschließend wurden 100µl 1:10 verdünnten Kulturüberstands des zu untersuchenden mAK gegen das gleiche Antigen zugegeben und für 30-60min bei RT inkubiert. Die Detektion des gebundenen Antikörpers erfolgte wie unter 3.4 beschrieben. Die niedrigste Konzentration, bei der noch eine Extinktion größer oder gleich dem zweifachen der Kontrolle detektiert wurde, wurde als Nachweisgrenze akzeptiert.

**Tabelle 1: Ergebnisse zur Charakterisierung der mAK**

| **Fusion/Klon** | **Ag-Spezifität** | **Isotyp** | **IB (Ag)** | **IB (HP-Lysat)** | **NWG (ng Ag/ml)** |
|---|---|---|---|---|---|
| HP8m/4H5-D4-C9 | β-Urease | IgG1, κ | +/- | +/- | 0,6 |
| HP9.1m/2D1-F6-G1 | | IgG1, κ | - | +/- | 0,3 |
| HP9.1 m/3C2-F8-E2 | | IgG1, κ | +/- | +/- | 1,2 |
| HP16.1m/3G1-H2-D7 | | IgG2a, κ | + | + | 0,6 |
| HP15m/3E8-D9-D6 | Alkylhydro- | IgG1, κ | + | + | 0,6 |
| HP15m/3F5-D5-B6 | peroxid- | IgG1, κ | + | + | 0,3 |
| | Reduktase | | | | |
| HP16m/2A5-E6-E5 | HSP60 | IgG1, κ | + | + | 3 |
| HP18.1m/3F11-G11-H11 | | IgG1,κ | + | + | 3 |
| HP18.1m/4D9-B9-A2 | | | | | |
| | | IgG1, κ | + | + | 3 |

| | | | | | |
|---|---|---|---|---|---|
| Abkürzungen: Ag Antigen; IB Immunblot; NWG Nachweisgrenze | | | | | |

### Ergebnisse

Tabelle 1 faßt die Ergebnisse der Isotypisierung, der Immunblotanalysen und der Nachweisgrenzenbestimmung für die in Tabelle 1 aufgelisteten mAK zusammen.

**Tabelle 2: Ergebnisse der Epitop-Kartierung monoklonaler Antikörper gegen H. pylori-Antigene**

| **Ag-Spezifität** | **Fusion/Klon** | **Epitop (AS-Position)** | **AS-Sequenz** |
|---|---|---|---|
| β-Urease | HP8m/1H5-G2-B4 | negativ | |
| (große Untereinheit) | HPBm/4H5-D4-C9 | negativ | |
| | HP9m/2B12-G7-B12 | 369-375 | VGEVITR |
| α-Urease | HP9m/2E7-B8-G10 | negativ | |
| (kleine Untereinheit) | HP9m/1H7-C6-D5 | negativ | |
| Alkylhydroperoxid- | HP15m/3E8-D9-D6 | negativ | |
| Reduktase | HP15m/3F5-D5-B6 | negativ | |
| | HP15m/4H12-A4- | negativ | |
| | D5 | 143-149 | LPLGRNA |
| | HP15m/1H7-H3-B7 | | |
| HSP60 | HP16m/2A5-E6-E5 | negativ | |
| | HP16m/1D10-A8 | negativ | |

| | | | |
|---|---|---|---|
| Abkürzungen: Ag Antigen; AS Aminosäure; negativ: Epitop konnte nicht kartiert werden | | | |

Bei zwei anti-α-Urease mAK und zwei anti-HSP60 mAK konnte mit der Immunblot-Methode kein Epitop kartiert werden. Von drei gegen die β-Urease gerichteten und vier gegen die Alkylhydroperoxid-Reduktase gerichteten mAK konnte nur bei je einem der mAK das Epitop bestimmt werden (siehe Tabelle 2). Diese Methode schien daher nicht geeignet, eventuelle Überlappungen der Epitope von Antikörpern gleicher Spezifität vorauszusagen.

### Beispiel 7: Charakterisierung von Antikörper-Antigen-Wechselwirkungen mittels Oberflächenplasmonresonanz-Spektroskopie

Die Epitop-Überlappungsmessung mit der SPR-Spektroskopie liefert Informationen über die gleichzeitige Zugänglichkeit von Antikörper-Epitopen. Damit lassen sich geeignete Antikörper-Pärchen für die Entwicklung von ELISA und Schnelltest finden (Fägerstam LG et al., 1990; Malmqvist M., 1996).

### Durchführung der Oberflächenplasmonresonanz-Spektroskopie am Pharmacia BIAcore

Alle Schritte wurden auf einer Pharmacia Biacore Processing Unit CA 186 nach Standardprotokollen durchgeführt (NHS/EDC, BIAcore Methods Manual). Hierzu wurde zunächst ein polyklonaler Kaninchen-anti-Maus-Antikörper auf der Dextranmatrix des BIAcore CM5 Glasträgerchips immobilisiert. Durch Zugabe des ersten *H. pylori*-antigenspezifischen monoklonalen Antikörpers (1. mAK; 100µg/ml; 10µl) wurde dieser mit dem immobilisiertem Fängerantikörper zur Reaktion gebracht und die entsprechende Veränderung am Detektor gemessen (ΔRU₁; RU Resonance Units). Nach Zugabe von *H. pylori*-Antigen (45µg/ml; 5µl) reagierte dies mit dem 1. mAK. Anschließend wurde verbliebener freier Fängerantikörper mit einem unspezifischen Mausantikörper (1mg/ml; 10µl) sowie oligomere Epitope durch Hinzufügen weiteren 1. mAK (100µg/ml; 10µl) geblockt. Nach der nun folgenden Reaktion des zweiten *H. pylori*-antigenspezifischen Antikörpers (2. mAK; 100µg/ml; 10µl) mit dem gebundenem Antigen wurde erneut die Veränderung im Signal des Detektors bestimmt (ΔRU₂) und zu der Veränderung am Detektor nach Zugabe des 1. mAK ins prozentuale Verhältnis gesetzt (ΔRU₂/ΔRU₁). Ein ΔRU₂/ΔRU₁<10% deutete auf überlappende Epitope hin. (Als überlappend werden die Epitope eines Antigens dann bezeichnet, wenn ein erster Antikörper alle von ihm zu bindenden Epitope eines Antigens besetzt hat und die nachfolgende Bindung eines zweiten Antikörpers be- bzw. verhindert). Durch kinetische Messungen lassen sich weiterhin die Werte für Geschwindigkeitskonstanten der Adsorption und Desorption von Antikörpern errechnen.

### Ergebnisse

Es wurden zwölf monoklonale anti-Urease-Antikörper und fünf Antikörper gegen Alkylhydroperoxid-Reduktase getestet. Durch Vergleich der 144 bzw. 25 Kombinationsmöglichkeiten untereinander wurden diejenigen Kombinationen bestimmt, die eine gleichzeitige Zugänglichkeit der Epitope zeigten und deren Affinitätskonstanten für die Verwendung im Stuhl-ELISA günstig erschienen. Tabelle 3 zeigt eine Auswahl der Ergebnisse aus der Kombination verschiedener Antikörper gegen Urease und stellt die kinetischen Konstanten der Eignung dieser Antikörper-Kombinationen im Stuhl-ELISA (Siehe Beispiel 9) gegenüber. Die Eignung von Antikörperpaaren für den ELISA muß sich hierbei nicht auf unabhängige Epitope beschränken. Auch eine Kombination aus Antikörpern, die z.T. überlappende Epitope erkennen (wie beispielsweise HP9.1 m-2D1 und HP8m-4H5), ist dadurch zu erklären, daß oligomere Proteine wie Urease ein gleiches Epitop mehrmals präsentieren. Dies ermöglicht im ELISA die Bindung eines Fang-Antikörpers an ein Epitop und die gleichzeitige Bindung eines oder mehrerer Detektions-Antikörper an ein oder mehrere gleiche Epitope des oligomeren Proteins.

**Tabelle 3: Vier mAK-Pärchen-Beziehungen für Urease bestimmt mittels Oberflächenplasmonenresonanz und Ihr Verhalten im ELISA**

| **Fänger** | **k**_{**off**} **/ k**_{**on**} | **Detektor** | **k**_{**off**} **/ k**_{**on**} | **Epitope** | **Bewertung im ELISA** |
|---|---|---|---|---|---|
| HP9.1m | 5,9*10⁻⁴/7,3*10⁴ | HP9.1m | 1,1*10⁻⁴/3,7*10⁴ | unabhängig | - |
| -2B11 | | -2D1 | | | |
| HP9.1m | 5,9*10⁻⁴/7,3*10⁴ | HP9.1m | 4,9*10⁻⁴/7,6*10⁴ | z.T. | - |
| -2B11 | | -3G1 | | überlappend | |
| HP9.1m | 1,1*10⁻⁴/3,7*10⁴ | HP8m | 4,5*10⁻⁶/4,8*10⁴ | z.T. | +++ |
| -2D1 | | -4H5 | | überlappend | |
| HP9.1m | 3,0*10⁻⁴/1,5*10⁵ | HP9.1m | 4,9*10⁻⁴/7,6*10⁴ | unabhängig | ++ |
| -XG1 | | -3G1 | | | |
| HP8m | 4,5*10⁻⁶/4,8*10⁴ | HP9.1 m | 2,6*10⁻⁴/7,4*10⁴ | unabhängig | +++ |
| -4H5 | | -3C2 | | | |

### Beispiel 8: Auswahl von Antikörperpaaren für die Verwendung im ELISA am menschlichen Stuhl

Bei denjenigen Antikörpern, welche die niedrigsten Nachweisgrenzen bei der Messung aus dem Kulturüberstand zeigten, wurden mittels Oberflächenplasmonresonanz Epitopüberlappungen bestimmt. Die Kombinationen, die sich bei diesen Messungen vielversprechend zeigten (möglichst geringe Epitopüberlappung, hohe Geschwindigkeitskonstante für Adsorption, niedrige Geschwindigkeitskonstante der Desorption) wurden auf ihre Nachweisgrenze im Stuhl-ELISA getestet.

### Beispiel 9: Detektion von H. pylori im menschlichen Stuhl mittels ELISA

Abkürzungen: Detektor: Detektions-Antikörper; Fänger: Fang-Antikörper

Als Testproben standen Stuhlproben von Patienten aus dem Klinikum Großhadern, München, zur Verfügung, deren Infektionsstatus mit *H. pylori* (Kategorien 0 und 4) mittels serologischer und histologischer Untersuchungen erhoben wurde. In die Kategorie 0 wurden Patienten eingeteilt, bei denen das klinische Ergebnis aus Serologie und Histologie eindeutig negativ war, bei Patienten der Kategorien 4 das klinische Ergebnis aus Serologie und Histologie eindeutig positiv war.

Zwischen den einzelnen Arbeitsschritten wurde die ELISA-Platte 2-3x mit 250µl PBS unter Zusatz von 0,025% (Waschpuffer 1) bzw. 0,2% Tween 20 (Waschpuffer2; VoL:Vol.) gewaschen. Die Beschichtung der ELISA-Platten (MaxiSorb; Nunc) erfolgte für 1 h bei 37ºC mit 100 µl einer mAK-Lösung (2.5 µg Antikörper/ml Carbonatpuffer, 0, 1 M, pH9,5). Zur Blockade der noch freien Bindungsstellen wurden 200µl 150mM PBS mit 0,2% Fischgelatine oder 1 % Magermilchpulver (Gew.:Vol.) pro Napf pipettiert und 30min bei Raumtemperatur inkubiert. Gewaschen wurde mit Waschpuffer 1. Humanstuhl wurde im Verhältnis 1:10 (Gew.:Vol.) mit 150mM PBS unter Zusatz von 2% Magermilchpulver suspendiert, gereinigte *H. pylori*-Antigene gelöst in 150mM PBS in bekannten Konzentrationen zugegeben. Je 100µl der Suspensionen wurden pro Napf für 1h inkubiert. Die Platte wurde zuerst von Hand gespült und 4x mit Waschpuffer 2 gewaschen. Anschließend wurden 100µl einer Lösung mit Biotin-gekoppelten mAK (0.5 µg Antikörper/ml PBS) gegen das gleiche Antigen zugegeben und für 30-60min bei RT inkubiert. Die Detektion der gebundenen Antigene erfolgt durch Zugabe eines Konjugats von Streptavidin mit POD (DAKO). Die POD setzt das farblose Substrat TMB (Sigma) in ein blaues Produkt um. Nach 5 bis 10 Minuten, oder sobald auch die Negativkontrolle eine leichte Blaufärbung zeigte, wurde die Reaktion durch Zugabe von 1 N Schwefelsäure (100 µl/Napf) gestoppt. Die Stärke der Farbreaktion wurde im ELISA-Reader (MWG Spektral) gemessen. Die Messung erfolgt bei 455 nm gegen die Referenzwellenlänge 620 nm.

**Tabelle 4:** Nachweis von *H. pylori*-Antigenen aus dem Stuhl sicher negativer (Kategorie 0) bzw. sicher positiver (Kategorie 4) Patienten mittels ELISA unter Verwendung monoklonaler Antikörper:

**Tabelle 4a: Kategorie 0 Proben: 0 bedeutet falsch-positiv, 1 bedeutet richtig-negativ**

| | **[µg Ag/ g Stuhl]** | | | **resultierender Wahrheitswert** | | | |
|---|---|---|---|---|---|---|---|
| **Probe** | **Urease** | **26kDa** | **HSP60** | **Urease** | **26kDa** | **HSP60** | **3-Kombination** |
| **cut-off** | | | | **1** | **15** | **5** | |
| 0047 | 0,6 | 2,3 | | 1 | 1 | | 1 |
| 0048 | 0,6 | 2,3 | | 1 | 1 | | 1 |
| 0051 | 2,1 | 22 | | 0 | 0 | | 0 |
| 0057 | 0,6 | 2,3 | | 1 | 1 | | 1 |
| 0069 | 0,6 | 2,3 | | 1 | 1 | | 1 |
| 0074 | 0,6 | 2,3 | | 1 | 1 | | 1 |
| 0087 | 0,6 | 2,3 | | 1 | 1 | | 1 |
| 0099 | 0,6 | 2,3 | | 1 | 1 | | 1 |
| 0185 | 0,6 | 2,3 | | 1 | 1 | | 1 |
| 0186 | 0,6 | 2,3 | | 1 | 1 | | 1 |
| 0189 | 0,6 | 2,3 | | 1 | 1 | | 1 |
| 0265 | 0,6 | 2,3 | | 1 | 1 | | 1 |
| 0298 | 0,6 | 2,3 | | 1 | 1 | | 1 |
| 0305 | 0,6 | 2,3 | | 1 | 1 | | 1 |
| 0373 | 0,6 | 2,3 | | 1 | 1 | | 1 |
| 0074 | 0,6 | 2,3 | 2,3 | 1 | 1 | 1 | 1 |
| 0089 | 0,6 | 2,3 | 2,3 | 1 | 1 | 1 | 1 |
| 0090 | 0,6 | 2,3 | 2,3 | 1 | 1 | 1 | 1 |
| 0091 | 0,76 | 2,3 | 2,3 | 1 | 1 | 1 | 1 |
| 0097 | 1,6 | 2,3 | 2,3 | 0 | 1 | 1 | 0 |
| 0103 | 1 | 2,4 | 15 | 1 | 1 | 0 | 0 |
| 0107 | 1 | 12 | 2,3 | 1 | 1 | 1 | 1 |
| 0114 | 4 | 2,3 | 44 | 0 | 1 | 0 | 0 |
| 0126 | 0,6 | 12 | 2,3 | 1 | 1 | 1 | 1 |
| 0130 | 0,76 | 67 | 2,3 | 1 | 0 | 1 | 0 |
| 0141 | 20 | 220 | 105 | 0 | 0 | 0 | 0 |
| 0142 | 1,8 | 2,3 | 2,3 | 0 | 1 | 1 | 0 |
| 0144 | 0,6 | 2,3 | 2,3 | 1 | 1 | 1 | 1 |
| 0146 | 0,6 | 2,3 | 2,3 | 1 | 1 | 1 | 1 |
| 0148 | 0,6 | 2,3 | 2,3 | 1 | 1 | 1 | 1 |
| 0168 | 0,6 | 2,3 | 2,3 | 1 | 1 | 1 | 1 |
| 0193 | 0,6 | 2,3 | 20 | 1 | 1 | 0 | 0 |
| 0207 | 0,6 | 2,3 | 20 | 1 | 1 | 0 | 0 |
| 0220 | 0,6 | 2,3 | 2,2 | 1 | 1 | 1 | 1 |
| 0225 | 0,6 | 2,3 | 2,2 | 1 | 1 | 1 | 1 |
| 0231 | 0,6 | 2,3 | 2,2 | 1 | 1 | 1 | 1 |
| 0235 | 0,6 | 2,3 | 2,2 | 1 | 1 | 1 | 1 |
| 0251 | 0,6 | 2,3 | | 1 | 1 | | 1 |
| 0258 | 0,6 | 95 | | 1 | 0 | | 0 |
| 0274 | 0,6 | 2,3 | | 1 | 1 | | 1 |

**Tabelle 4b: Kategorie 4 Proben: 0 bedeutet falsch-negativ, 1 bedeutet richtig-positiv**

| | **[µg Ag/ g Stuhl]** | | | **resultierender Wahrheitswert** | | | |
|---|---|---|---|---|---|---|---|
| **Probe** | **Urease** | **26kDa** | **HSP60** | **Urease** | **26kDa** | **HSP60** | **3-Kombination** |
| **cut-off** | | | | **1** | **15** | **5** | |
| 0006 | 1,9 | 25 | 34 | 1 | 1 | 1 | 1 |
| 0010 | 5,6 | 91,5 | 170 | 1 | 1 | 1 | 1 |
| 22 | 7,8 | 45 | 25 | 1 | 1 | 1 | 1 |
| 0350 | 15,6 | 198 | 95 | 1 | 1 | 1 | 1 |
| 0288 | 18 | 34 | 65,9 | 1 | 1 | 1 | 1 |
| 0332 | 29 | 360 | 650 | 1 | 1 | 1 | 1 |
| 0218 | 7 | 24 | 3 | 1 | 1 | 0 | 1 |
| 0285 | 7,4 | 115 | 3 | 1 | 1 | 0 | 1 |
| 0004 | 9,5 | 2,3 | 350 | 1 | 0 | 1 | 1 |
| 11 | 21 | 15 | 26 | 1 | 0 | 1 | 1 |
| 8 | 105 | 2,3 | 89 | 1 | 0 | 1 | 1 |
| 0003 | 1,5 | 2,3 | 10 | 1 | 0 | 1 | 1 |
| 15 | 2,3 | 2,3 | 15 | 1 | 0 | 1 | 1 |
| 3005 | 1,2 | 2,3 | 2,3 | 1 | 0 | 0 | 1 |
| 0002 | 1,8 | 2,3 | 2,3 | 1 | 0 | 0 | 1 |
| 0206 | 9,6 | 2,9 | 3 | 1 | 0 | 0 | 1 |
| 0135 | 11 | | 3 | 1 | 0 | 0 | 1 |
| 0175 | 16 | 2,9 | 3 | 1 | 0 | 0 | 1 |
| 0005 | 25 | 2,3 | 2,3 | 1 | 0 | 0 | 1 |
| 0278 | 25,7 | 4 | 3 | 1 | 0 | 0 | 1 |
| 0001 | 127 | 2,3 | 2,3 | 1 | 0 | 0 | 1 |
| 0004 | 127 | 2,9 | 3 | 1 | 0 | 0 | 1 |
| 0037 | 127 | 2,9 | 3 | 1 | 0 | 0 | 1 |
| 0294 | 127 | 14 | 2,3 | 1 | 0 | 0 | 1 |
| 0108 | 17,5 | | 3 | 1 | | 0 | 1 |
| 28 | 0,6 | 69 | 330 | 0 | 1 | 1 | 1 |
| 0012 | 0,6 | 23 | 8,6 | 0 | 1 | 1 | 1 |
| 0013 | 0,6 | 27,7 | 3 | 0 | 1 | 0 | 1 |
| 6c | 0,6 | 2,8 | 35 | 0 | 0 | 1 | 1 |
| 18c | 0,6 | 2,3 | 15 | 0 | 0 | 1 | 1 |
| 53 | 0,6 | 2,3 | 17 | 0 | 0 | 1 | 1 |
| 0164 | 0,6 | 2,9 | 5,9 | 0 | 0 | 1 | 1 |
| 3007 | 0,6 | 2,3 | 2,3 | 0 | 0 | 0 | 0 |
| 9c | 0,6 | 2,3 | 2,3 | 0 | 0 | 0 | 0 |
| 13c | 0,6 | 2,3 | 2,3 | 0 | 0 | 0 | 0 |
| 47 | 0,6 | 2,3 | 2,3 | 0 | 0 | 0 | 0 |
| 17 | 0,6 | 2,3 | 2,3 | 0 | 0 | 0 | 0 |
| 18 | 0,6 | 2,3 | 2,3 | 0 | 0 | 0 | 0 |
| 0331 | 0,6 | 2,3 | 3 | 0 | 0 | 0 | 0 |
| 0063 | 0,6 | 2,9 | 3 | 0 | 0 | 0 | 0 |
| 0179 | 0,6 | 2,9 | 3 | 0 | 0 | 0 | 0 |
| 0213 | 0,6 | 2,9 | 3 | 0 | 0 | 0 | 0 |
| 0233 | 0,6 | 2,9 | 3 | 0 | 0 | 0 | 0 |
| 0081 | 0,6 | 2,9 | 14,4 | 0 | | 1 | 1 |

**Tabelle 4c: Wahrheitswerte für den Nachweis von Urease, 26kDa-Protein, und HSP60**

| | **Wahrheitswert** | | | |
|---|---|---|---|---|
| | **Urease** | **26kDa** | **HSP60** | **3-Kombination** |
| **Schwellenwert** | **1** | **15** | **5** | |
| **Spezifität** | **88%** | **90%** | **77%** | **75%** |
| **Sensitivität** | **57%** | **26%** | **41%** | **75%** |
| negativ-richtige | 35 | 36 | 17 | 30 |
| negativ-gesamt | 40 | 40 | 22 | 40 |
| positiv-richtige | 25 | 11 | 18 | 33 |
| positiv-gesamt | 44 | 42 | 44 | 44 |

Abkürzungen: 26kDa bedeutet 26 kDa-Protein; 3-Kombination bedeutet Kombination aller drei Antigene (Urease, 26kDa-Protein und HSP60)

Als Fänger-Antikörper und Detektions-Antikörper wurden bei Urease HP8m/4H5 + HP16m/XG1 eingesetzt. Die Nachweisgrenze lag bei 0,075 ng/ml. Bei 26kDa-Protein wurden als Fänger-Antikörper HP15m/4H12 und als Detektions-Antikörper HP15m/3E8 eingesetzt. Die Nachweisgrenze lag bei 1,5 ng/ml. Bei HSP60 wurden als Fang-Antikörper HP18.1m/3F11 und als Detektions-Antikörper HP16m/2A5 + HP18.1 m/4D9 eingesetzt. Die Nachweisgrenze lag bei 6 ng/ml.

### Ergebnisse

Die Tabellen 4a - 4c zeigen die Ergebnisse der Untersuchung von Stuhlproben für Urease, Alkylhydroperoxid-Reduktase und HSP60. Die letzte Spalte (3-Kombination) zeigt jeweils die Werte für den resultierenden Wahrheitswert der Kombination aus allen drei Antigenen (Urease, 26kDa-Protein, HSP60).

Insgesamt wurden von 40 untersuchten Kategorie 0 Kontrollproben (Tabelle 4a) 30 als negativ identifiziert (Spezifität 75%), von 44 Kategorie 4 Proben (Tabelle 4b) wurden 33 als positiv identifiziert (Sensitivität 75%). In 6 von 44 Proben war der Nachweis von allen drei Antigenen möglich, in 25 von 44 Proben konnte Urease, in 11 von 42 Proben konnte Alkylhydroperoxid-Reduktase und in 18 von 44 Proben konnte HSP60 nachgewiesen werden. Für die Kombination aus allen drei Antigenen (Urease, 26kDa, HSP60) wurde somit eine Spezifität von 75% sowie eine Sensitivität von 75% erreicht (Tabelle 4c).

Tabelle 5 zeigt das Ergebnis der Untersuchung von 3 Proben, die aus dem Stuhl eines Patienten an drei verschiedenen Stellen entnommen wurden. Die Zahlenangaben entsprechen der gemessenen Extinktion bei 650nm dividiert durch die Extinktion einer Nullprobe, also dem Vielfachen des Hintergrunds (H). Es zeigte sich eine inhomogene Verteilung der nachgewiesenen Antigene.

**Tabelle 5: Verteilung H. pylori-spezifischer Antigene in Stuhlproben, die an verschiedenen Stellen des Stuhlgangs eines Infizierten entnommen wurden**

| **Detektiertes Antigen** | **β-Urease** | **26kDa-Protein** | **HSP60** |
|---|---|---|---|
| Probe 1 | 8,6 x H | 2,5 x H | 1 x H |
| Probe 2 | 11 x H | 1 x H | 1 x H |
| Probe 3 | 3,5 x H | 1 x H | 1 x H |

### Beispiel 10: Klonierung und Sequenzbestimmung der funktionellen variablen Bereiche von Immunoglobulinen aus Hybridom-Zellinien

Gesamt-RNA wurde aus Antikörper-produzierenden Hybridom-Zellinien nach Chomczynski (Chomczynski, 1987) isoliert.

Anschließend wurde die entsprechende cDNA nach Standard-Methoden synthetisiert (Sambrook et al., 1989)

Die DNA-Regionen, welche die kappa-leichte Kette sowie das schwere Kette-Fd-Segment (VH + CH1) der jeweiligen Antikörper kodieren wurden mittels PCR amplifiziert. Dabei kam das in Tabelle 6 aufgeführte Oligunukleotid-Primer Set zur Anwendung. Die aus den einzelnen Hybridom-Zellinien isolierte cDNA diente als Matrize.

Das eingesetzte Primer-Set führt zu je einer 5'-*XhoI* und einer 3'-*Spe*I Schnittstelle in den schwere Kette-Fd-Fragmenten sowie zu je einer 5'-*Sac*I und einer *3'-Xba*l-Schnittstelle in den kappa-leichten Ketten. Zur PCR-Amplifikation der schwere Kette-Fd-kodierenden DNA-Fragmente wurden 11 verschiedene 5'-VH-Primer (MVH 1-8 und MULH1-3) jeweils kombiniert mit dem 3'-VH-Primer MlgG1. Zur Amplifikation der DNA-Fragmente, welche für die kappa leichten Ketten kodieren, wurden 11 verschiedene 5'-VK-Primer (MUVK 1-7 und MULK1-4) jeweils kombiniert mit dem 3'-VK-Primer 3'MUCK.

Das folgende Temperaturprogramm kam bei allen PCR-Amplifikationen zur Anwendung: anfängliche Denaturierung bei 94°C für 3 min, Denaturierung bei 94 °C für 25 s, primer-Anlagerung bei 52°C für 60 s, Polymerisation bei 72 °C für 90 s. Dieses Programm wurde für 40 Zyklen beibehalten, gefolgt von einer 10 min. abschließenden Vervollständigung der Fragmente bei 72 °C.

Die Ergebnisse der PCR-Amplifikationen wurden mittels Agarose-Gel-Elektrophorese aufgetrennt und DNA-Banden des erwarteten Molekulargewichts isoliert. Die isolierten Banden wurden anschließend einem Restriktionsverdau unter Einsatz der Enzyme *Xhol* und *Spel* (schwere Ketten) bzw. *Sacl* und *Xbal* (leichte Ketten) unterzogen und die erhaltenenen Fragmente in den Plasmid-Vektor Bluescript KS (Stratagene) kloniert, nachdem dieser zunächst mit den Restriktionsenzymen *Xhol* und *Spel* bzw. *Sacl* und *Xbal* gespalten worden war.

Plasmid-Präparationen der klonierten schwere und leichte Ketten-Fragmente wurden daraufhin sequenzanalysiert. Für jede Hybridom-Zellinie wurden Sequenzen ausgewählt, die für funktionelle variable Bereiche der Immunglobulin schwere und leichte Kette (VH bzw. VL) kodieren. Auf diese Weise konnte für jede Hybridom-Zellinie genau ein funktioneller VH- und ein funktioneller VL-Bereich identifiziert werden. Die funktionellen VH- und VL-Sequenzen sind in Abbildungen 1-8 wiedergegeben. Klonierung und Sequenzierung wurden nach Standardmethoden durchgeführt (Sambrook et al., 1989).

**Tabelle 6: Liste der für die PCR-Amplifikation der funktionellen variablen Bereiche von schweren und leichten Immunglobulin-Ketten verwendet wurden**

| | |
|---|---|
| MVH1 | (GC)AG GTG CAG CTC GAG GAG TCA GGA CCT |
| MVH2 | GAG GTC CAG CTC GAG CAG TCT GGA CCT |
| MVH3 | CAG GTC CAA CTC GAG CAG CCT GGG GCT |
| MVH4 | GAG GTT CAG CTC GAG CAG TCT GGG GCA |
| MVH5 | GA(AG) GTG AAG CTC GAG GAG TCT GGA GGA |
| MVH6 | GAG GTG AAG CTT CTC GAG TCT GGA GGT |
| MVH7 | GAA GTG AAG CTC GAG GAG TCT GGG GGA |
| MVH8 | GAG GTT CAG CTC GAG CAG TCT GGA GCT |
| MULK1 | GGG GAG CTC CAC CAT GGA GAC AGA CAC ACT CCT GCT AT |
| MULK2 | GGG GAG CTC CAC CAT GGA TTT TCA AGT GCA GAT TTT CAG |
| MULK3 | GGG GAG CTC CAC CAT GGA GWC ACA KWC TCA GGT CTT TRT A |
| MULK4 | GGG GAG CTC CAC CAT GKC CCC WRC TCA GYT YCT KGT |
| MIgG1 | TAT GCA ACT AGT ACA ACC ACA ATC CCT GGG |
| MUVK1 | CCA GTT CCG AGC TCG TTG TGA CTC AGG AAT CT |
| MUVK2 | CCA GTT CCG AGC TCG TGT TGA CGC AGC CGC CC |
| MUVK3 | CCA GTT CCG AGC TCG TGC TCA CCC AGT CTC CA |
| MUVK4 | CCA GTT CCG AGC TCC AGA TGA CCC AGT CTC CA |
| MUVK5 | CCA GAT GTG AGC TCG TGA TGA CCC AGA CTC CA |
| MUVK6 | CCA GAT GTG AGC TCG TCA TGA CCC AGT CTC CA |
| MUVK7 | CCA GTT CCG AGC TCG TGA TGA CAC AGT CTC CA |
| MULH1 | GGG CTC GAG CAC CAT GGR ATG SAG CTG KGT MAT SCT CTT |
| MULH2 | GGG CTC GAG CAC CAT GRA CTT CGG GYT GAG CTK GGT TTT |
| MULH3 | GGG CTC GAG CAC CAT GGC TGT CTT GGG GCT GCT CTT CT |
| 3'MUCK | GCG CCG TCT AGA ATT AAC ACT CAT TCC TGT TGA A |

### Literatur:

**Coller & Coller, 1983:** Coller, H.A., Coller, B.S., Meth. Enzymol. **121**:412-417

**Harlow & Lane, 1988:** Harlow, E., Lane, D., Antibodies: A laboratory manual, Cold Spring Harbour Laboratory, New York

**Kearney et al., 1979:** Kearney, J. Immunol. **123:** 1548-1550

**Laemmli, 1970:** Laemmli, E.K., Nature **227:** 680-685

**Peters & Baumgarten, 1990:** Peters, J.H., Baumgarten, H. (Hrsg.), Monoklonale Antikörper, Springer Verlag, Berlin

**Fägerstam et al., 1990:** Fägerstam, L.G. et al., J. Mol. Recognit. **3:** 208-214

**Malmqvist, 1996: Malmqvist, M.,** Methods **9**: 525-532

**Eschweiler et al., 1993:** Eschweiler, B., et al., Zentralbl. F. Bakt. **280:** 73-85

**Pharmacia Biotech, 1994:** Monoclonal Antibody Purification Handbook

**Chomczynski, 1987:** Anal. Biochem. **162**: 156-159

**Sambrook et al., 1989:** Molecular cloning: A laboratory manual, Cold Spring Harbour Laboratory Press, second edition

**Vaughan et al., 1998:** Nature Biotechnology **16:** 535-539

**Orlandi et al., 1989:** Proc. Natl. Acad. Sci USA **86:** 3833-3837

**Janeway & Travers, 1997:** Immunologie, 2. Auflage, Spektrum Akademischer Verlag GmbH, Heidelberg

**Osborne et al., 1997:** Curr. Opin. Chem. Biol. **1**: 5-9

**Stull und Szoka, 1995**: Pharm. Res. **12:** 465-483

## Patentansprüche

1. Verfahren zum Nachweis einer Infektion eines Säugers mit einem Säure-resistenten Mikroorganismus, wobei man
(a) eine Stuhlprobe des Säugers mit zwei oder drei verschiedenen monoklonalen Antikörpern, Fragmenten oder Derivaten davon oder Aptameren unter Bedingungen inkubiert, die eine Komplexbildung von Antigenen aus dem Säure-resistenten Mikroorganismus mit den Antikörpern, Fragmenten oder Derivaten davon oder den Aptameren erlauben, und wobei
(aa) der erste monoklonale Antikörper oder das Fragment oder Derivat davon oder das erste Aptamer ein Epitop des ersten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid das eine Sequenz des Proteins oder Fragments aufweist, Antikörper produziert;
(ab) der zweite monoklonale Antikörper oder das Fragment oder Derivat davon oder das zweite Aptamer ein vom Epitop des ersten Antigens verschiedenes Epitop eines zweiten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid das eine Sequenz des Proteins oder Fragments aufweist, Antikörper produziert,
wobei die Teile der Säuger gemäß (aa) und gemäß (ab) überlappen können und in der Summe mehr als 70% der infizierten Säuger ausmachen; und
(b) die Bildung mindestens eines Antigen-Antikörperkomplexes oder Antigen-Aptamerkomplexes gemäß (aa) oder (ab) nachweist.

2. Verfahren nach Anspruch 1, wobei der Mikroorganismus ein Säure-resistentes Bakterium ist.

3. Verfahren nach Anspruch 2, wobei das Säure-resistente Bakterium ein Bakterium der Gattung Helicobacter oder der Gattung Mycobacterium oder der Gattung Campylobacter ist.

4. Verfahren nach Anspruch 3, wobei das Bakterium ein Bakterium der Spezies *Helicobacter pylori oder Helicobacter hepaticus* oder der Spezies *Mycobacterium tuberculosis* oder der Spezies *Campylobacter jejuni* oder *Campylobacter pylori* ist.

5. Verfahren nach einem der Ansprüche 1 bis 4, wobei das Epitop des ersten Antigens ein Epitop einer Urease und das Epitop des zweiten Antigens ein Epitop eines Hitzeschockproteins, einer Alkylhydroperoxid-Reduktase oder des 20kDa-Proteins (3-Dehydro-Quinase Typ II), des 16,9kDa-Proteins (Neutrophile-aktivierendes Protein) oder des 33,8 kDa-Proteins (Fruktose-Bisphosphat-Aldolase) ist.

6. Verfahren nach Anspruch 5, wobei die Urease die β-Urease von *H. pylori* ist.

7. Verfahren nach Anspruch 5 oder 6, wobei das Hitzeschockprotein ein Hsp60 ist.

8. Verfahren nach einem der Ansprüche 5 bis 7, wobei die Alkylhydroperoxid-Reduktase das 26kDa-Protein von *H. pylori* ist.

9. Verfahren insbesondere nach einem der Ansprüche 1 bis 8, wobei man
(a) eine Stuhlprobe des Säugers mit drei verschiedenen monoklonalen Antikörpern, Fragmenten oder Derivaten davon oder Aptameren unter Bedingungen inkubiert, die eine Komplexbildung von Antigenen aus dem Säure-resistenten Mikroorganismus mit den Antikörpern, Fragmenten davon oder Derivaten oder den Aptameren erlauben, und wobei
(aa) der erste monoklonale Antikörper oder das Fragment oder Derivat davon oder das erste Aptamer ein Epitop des ersten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert;
(ab) der zweite monoklonale Antikörper oder das Fragment oder Derivat davon oder das zweite Aptamer ein vom Epitop des ersten Antigens verschiedenes Epitop eines zweiten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert; und
(ac) der dritte monoklonale Antikörper oder das Fragment oder Derivat davon oder das dritte Aptamer ein vom Epitop des ersten und zweiten Antigens verschiedenes Epitop eines dritten Antigens spezifisch bindet, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert,
wobei die Teile der Säuger gemäß (aa), gemäß (ab) und gemäß (ac) überlappen können und in der Summe im wesentlichen die Gesamtanzahl der infizierten Säuger ausmachen, und
(b) die Bildung mindestens eines Antigen-Antikörperkomplexes oder eines Antigen-Aptamerkomplexes gemäß (aa), (ab) oder (ac) nachweist.

10. Verfahren nach Anspruch 9, wobei das Epitop des ersten Antigens ein Epitop einer Urease, vorzugsweise der β-Urease von *H. pylori,* das Epitop des zweiten Antigens ein Epitop eines Hitzeschockproteins, vorzugsweise von Hsp60 aus *H. pylori* und das Epitop des dritten Antigens ein Epitop einer Alkylhydroperoxid-Reduktase, vorzugsweise des 26kDa-Proteins von *H. pylori* ist oder wobei das Epitop des zweiten oder dritten Antigens ein Epitop einer Alkylhydroperoxid-Reduktase oder des 20kDa-Proteins (3-Dehydro-Quinase Typ II), des 16,9kDa-Proteins (Neutrophile-aktivierendes Protein) oder des 33,8 kDa-Proteins (Fruktose-Bisphosphat-Aldolase) ist.

11. Verfahren nach einem der Ansprüche 1 bis 10, wobei mindestens einer der monoklonalen Antikörper bzw. eines der Fragmente, Derivate oder Aptamere ein Konformationsepitop bindet.

12. Verfahren nach Anspruch 11, wobei sämtliche monoklonalen Antikörper bzw. Fragmente, Derivate oder Aptamere Konformationsepitope binden.

13. Verfahren zum Nachweis einer Infektion mit *Helicobacter pylori* im Stuhl eines Säugers, wobei man
(a) eine Stuhlprobe mit zwei verschiedenen monoklonalen Antikörpern, Fragmenten, Derivaten davon oder Aptameren unter Bedingungen inkubiert, die eine Antigen-AntikörperlAntigen-Aptamer-Komplexbildung erlauben, wobei
(aa) der erste monoklonale Antikörper, das Fragment, Derivat davon oder das erste Aptamer spezifisch β-Urease oder ein Fragment davon bindet;
(ab) der zweite monoklonale Antikörper, das Fragment, Derivat davon oder das zweite Aptamer spezifisch das 26kDa-Antigen oder ein Fragment davon bindet oder spezifisch Hsp60 oder ein Fragment davon bindet; und
(b) die Bildung mindestens eines Antigen-Antikörperkomplexes/Antigen-Aptamerkomlexes gemäß (aa) oder (ab) nachweist.

14. Verfahren zum Nachweis einer Infektion mit *Helicobacter pylori* im Stuhl eines Säugers, wobei man
(a) eine Stuhlprobe mit drei verschiedenen monoklonalen Antikörpern, Fragmenten, Derivaten davon oder Aptameren unter Bedingungen inkubiert, die eine Antigen-Antikörper/Antigen-Aptamer-Komplexbildung erlauben, wobei
(aa) der erste monoklonale Antikörper, das Fragment, Derivat davon oder das erste Aptamer spezifisch β-Urease oder ein Fragment davon bindet;
(ab) der zweite monoklonale Antikörper, das Fragment, Derivat davon oder das zweite Aptamer spezifisch Hsp60 oder ein Fragment davon bindet; und
(ac) der dritte monoklonale Antikörper, das Fragment, Derivat davon oder das dritte Aptamer spezifisch das 26kDa-Antigen oder ein Fragment davon bindet; und
(b) die Bildung mindestens eines Antigen-Antikörper/Antigen-AptamerKomplexes gemäß (aa), (ab) oder (ac) nachweist.

15. Verfahren nach einem der Ansprüche 7 bis 14, wobei der Hsp60-spezifische Antikörper der von dem bei der Deutschen Sammlung von Mikroorganismen und Zellkulturen (DSMZ) am 23. Juni 1998 nach den Statuten des Budapester Vertrages unter der Hinterlegungsnummer DSM ACC2356 hinterlegten Hybridom HP16m/2A5-E6-E5 produzierte Antikörper ist.

16. Verfahren nach einem der Ansprüche 8 bis 15, wobei der 26kDa-Antigen-spezifische Antikörper der von dem bei der Deutschen Sammlung von Mikroorganismen und Zellkulturen (DSMZ) am 23. Juni 1998 nach den Statuten des Budapester Vertrages unter der Hinterlegungsnummer DSM ACC2355 hinterlegten Hybridom HP15m/3E8-D9-D6 produzierte Antikörper ist.

17. Verfahren nach einem der Ansprüche 6 bis 16, wobei der β-Ureasespezifische Antikörper der von einem der bei der Deutschen Sammlung von Mikroorganismen und Zellkulturen (DSMZ) am 23. Juni 1998 nach den Statuten des Budapester Vertrages unter den Hinterlegungsnummem DSM ACC2360 oder DSM ACC2362 hinterlegten Hybridomen HP8m/4H5-D4-C9 oder HP9.1 m/3C2-F8-E2 produzierte Antikörper ist.

18. Verfahren nach einem der Ansprüche 7 bis 17, wobei die schwere Kette des ein Hsp60-Epitop bindenden Antikörpers mindestens eine der folgenden CDRs, vorzugsweise die CDR3 und weiter bevorzugt alle drei folgenden CDRs aufweist:
CDR1: GFSLSRYSVH
CDR2: MIWGGGSTDYNSGLKS
CDR3: NMGGRYPDYFDY

19. Verfahren nach Anspruch 18, wobei die die schwere Kette des Antikörpers kodierende DNA-Sequenz mindestens eine der folgenden, CDRs kodierenden DNA-Sequenzen, vorzugsweise die die CDR3 kodierende DNA-Sequenz und weiter bevorzugt alle drei folgenden, CDRs kodierenden DNA-Sequenzen aufweist:
CDR1: GG GTTCTCATTA TCCAGATATA GTGTACAC
CDR2: ATGATATGGG GTGGTGGAAG CACAGACTAT AATTCAGGTC TCAAATCC
CDR3: AATATG GGGGGTAGGT ACCCGGACTA CTTTGACTAC

20. Verfahren nach einem der Ansprüche 7 bis 19, wobei die leichte Kette des ein Hsp60-Epitop bindenden Antikörpers mindestens eine der folgenden CDRs, vorzugsweise die CDR3 und weiter bevorzugt alle drei folgenden CDRs aufweist:
CDR1: RASKSVSTSGYSYIH
CDR2: LASNLES
CDR3. QHSRELPLT

21. Verfahren nach Anspruch 20, wobei die die leichte Kette des Antikörpers kodierende DNA-Sequenz mindestens eine der folgenden, CDRs kodierenden DNA-Sequenzen, vorzugsweise die die CDR3 kodierende DNA-Sequenz und weiter bevorzugt alle drei folgenden, CDRs kodierenden DNA-Sequenzen aufweist:
CDR1: A GGGCCAGCAA GAGTGTCAGT ACATCTGGCT ATAGTTACAT ACAC
CDR2: C TTGCATCCAA CCTAGAATCT
CDR3: CAGC ACAGTAGGGA GCTTCCGCTC ACG.

22. Verfahren nach einem der Ansprüche 8 bis 21, wobei die schwere Kette des ein 26kDa-Protein bindenden Antikörpers mindestens eine der folgenden CDRs, vorzugsweise die CDR3 und weiter bevorzugt alle drei folgenden CDRs aufweist:
CDR1: GFTFNSYAMY
CDR2: RIRSKSDNYATYYANSVKD
CDR3: DHDKFPFYYALDY

23. Verfahren nach Anspruch 22, wobei die die schwere Kette des Antikörpers kodierende DNA-Sequenz mindestens eine der folgenden, CDRs kodierenden DNA-Sequenzen, vorzugsweise die die CDR3 kodierende DNA-Sequenz und weiter bevorzugt alle drei folgenden, CDRs kodierenden DNA-Sequenzen aufweist:
CDR1: GG TTTCACCTTC AATTCCTATG CCATGTAC
CDR2: CGCATAAGAA GTAAAAGTGA TAATTATGCA ACATATTATG CCAATTCAGT GAAAGAC
CDR3: GATCATG ATAAGTTTCC TTTTTACTAT GCTCTGGACT AC

24. Verfahren nach einem der Ansprüche 8 bis 23, wobei ein Antikörper, Fragment oder Derivat davon eingesetzt wird, dessen leichte Kette des ein 26kDa-Protein bindenden Antikörpers mindestens eine der folgenden CDRs, vorzugsweise die CDR3 und weiter bevorzugt alle drei folgenden CDRs aufweist:
CDR1: TASSSVSSSYLH
CDR2: STSNLAS
CDR3: HQYHRSPPT

25. Verfahren nach Anspruch 24, wobei die die leichte Kette des Antikörpers kodierende DNA-Sequenz mindestens eine der folgenden, CDRs kodierenden DNA-Sequenzen, vorzugsweise die die CDR3 kodierende DNA-Sequenz und weiter bevorzugt alle drei folgenden, CDRs kodierenden DNA-Sequenzen aufweist:
CDR1: A CTGCCAGCTC AAGTGTGAGT TCCAGTTACT TGCAC
CDR2: AGCACTTCCA ACCTGGCTTC T
CDR3: CAC CAGTATCATC GTTCCCCACC GACG

26. Verfahren nach einem der Ansprüche 6 bis 25, wobei die schwere Kette des ein Epitop der β-Urease bindenden Antikörpers mindestens eine der folgenden CDRs, vorzugsweise die CDR3 und weiter bevorzugt alle drei folgenden CDRs aufweist:
CDR1: GFTFSSHFMS
CDR2: SISSGGDSFYPDSLKG
CDR3: DYSWYALDY
oder:
CDR1: GYAFSTSWMN
CDR2: RIYPGDGDTNYNGKFKG
CDR3: EDAYYSNPYSLDY

27. Verfahren nach Anspruch 26, wobei die die schwere Kette kodierende DNA-Sequenzen des Antikörpers mindestens eine der folgenden, CDRs kodierenden DNA-Sequenz, vorzugsweise die die CDR3 kodierende DNA-Sequenz und weiter bevorzugt alle drei folgenden, CDRs kodierenden DNA-Sequenzen aufweist:
CDR1: GG CTACGCATTC AGTACCTCCT GGATGAAC
CDR2: CGGATTTATC CTGGAGATGG AGATACTAAC TACAATGGGA AGTTCAAGGG C
CDR3: GAG GATGCCTATT ATAGTAACCC CTATAGTTTG GACTAC
oder:
CDR1: GG ATTCACTTTC AGTAGCCATT TCATGTCT
CDR2: TCCATTAGTA GTGGTGGTGA CAGTTTCTAT CCAGACAGTC TGAAGGGC
CDR3: GACTAC TCTTGGTATG CTTTGGACTA C

28. Verfahren nach einem der Ansprüche 6 bis 27, wobei die leichte Kette des ein Epitop der β-Urease bindenden Antikörpers mindestens eine der folgenden CDRs, vorzugsweise die CDR3 und weiter bevorzugt alle drei folgenden CDRs aufweist:
CDR1: RASQSIGTRIH
CDR2: YGSESIS
CDR3: QQSNTWPLT
oder:
CDR1: HASQNINVWLS
CDR2: KASNLHT
CDR3: QQGRSYPLT

29. Verfahren nach Anspruch 28, wobei die die leichte Kette des Antikörpers kodierende DNA-Sequenz mindestens eine der folgenden, CDRs kodierenden DNA-Sequenzen, vorzugsweise die die CDR3 kodierende DNA-Sequenz und weiter bevorzugt alle drei folgenden, CDRs kodierenden DNA-Sequenzen aufweist:
CDR1: A GGGCCAGTCA GAGCATTGGC ACAAGAATAC AC
CDR2: TAT GGTTCTGAGT CTATCTCT
CDR3: CAACAA AGTAATACCT GGCCGCTCAC G
oder:
CDR1: C ATGCCAGTCA GAACATTAAT GTTTGGTTAA GC
CDR2: AAG GCTTCCAACT TGCACACA
CDR3: CAACAG GGTCGAAGTT ATCCTCTCAC G

30. Verfahren nach einem der Ansprüche 6 bis 29, wobei die Antikörper in den variablen Regionen der leichten und schweren Ketten die in den Figuren 1 und 2, den Figuren 3 und 4, den Figuren 5 und 6 oder den Figuren 7 und 8 dargestellten Aminosäuresequenzen aufweisen.

31. Verfahren nach einem der Ansprüche 6 bis 30, wobei die kodierenden Bereiche der variablen Regionen der leichten und schweren Ketten die in den Figuren 1 und 2, den Figuren 3 und 4, den Figuren 5 und 6 oder den Figuren 7 und 8 dargestellten DNA-Sequenzen aufweisen.

32. Verfahren nach einem der Ansprüche 1 bis 31, wobei mit der Stuhlprobe vor der Inkubation mit den Antikörpern folgende Schritte durchgeführt werden: (a) Resuspendieren der Stuhlprobe 1:3 bis 1:25, vorzugsweise etwa 1:10 in Resuspendierungspuffer und (b) Mischen auf einem Vortexmixer.

33. Verfahren nach einem der Ansprüche 1 bis 32, wobei der Nachweis der Bildung des mindestens einen Antigen-Antikörperkomplexes/Antigen-Aptamerkomplexes in Schritt (b) mittels eines immunologischen Verfahrens erfolgt.

34. Verfahren nach einem der Ansprüche 1 bis 33, wobei der Nachweis der Bildung des mindestens einen Antigen-Antikörperkomplexes/Antigen-Aptamerkomplexes in Schritt (b) mittels ELISA, RIA, Western Blot oder eines immunchromatographischen Verfahrens erfolgt.

35. Verfahren nach Anspruch 33 oder 34, wobei im RIA oder im ELISA der gleiche Antikörper oder dessen Fragment oder Derivat oder das gleiche Aptamer zur Bindung an die Festphase wie zum Nachweis des Epitops eingesetzt wird.

36. Verfahren nach einem der Ansprüche 1 bis 35, wobei die Antikörper, Fragmente oder Derivate davon oder die Aptamere an einem Träger fixiert sind.

37. Verfahren nach einem der Ansprüche 1 bis 36, wobei der monoklonale Antikörper ein Maus-Antikörper ist.

38. Verfahren nach Anspruch 36, wobei das Trägermaterial des Trägers ein poröses Trägermaterial ist.

39. Verfahren nach Anspruch 36 oder 38, wobei das Trägermaterial ein Teststreifen ist.

40. Verfahren nach Anspruch 36, 38 oder 39, wobei das Trägermaterial aus Zellulose oder einem Zellulosederivat besteht.

41. Verfahren nach einem der Ansprüche 1 bis 40, wobei der Säuger ein Mensch ist.

42. Monoklonaler Antikörper, Fragment oder Derivat davon, der/das eine V-Region aufweist, die eine Kombination der in einem der Ansprüche 18 bis 29 dargestellten CDRs aufweist oder der von einem der in den Ansprüchen 15 bis 17 dargestellten Hybridomen produziert wird.

43. Monoklonaler Antikörper, Fragment oder Derivat davon nach Anspruch 42, der/das mindestens eine der in den Figuren 1 bis 8 dargestellten V-Regionen aufweist.

44. Monoklonaler Antikörper, Fragment oder Derivat davon nach Anspruch 42 oder 43, der ein Maus-Antikörper oder ein Fragment oder Derivat davon oder ein chimärer, vorzugsweise ein humanisierter Antikörper oder ein Fragment oder Derivat davon ist.

45. Aptamer, das dasselbe Epitop wie der monoklonale Antikörper, das Fragment oder Derivat davon nach einem der Ansprüche 42 bis 44 spezifisch bindet.

46. Diagnostische Zusammensetzung enthaltend mindestens zwei monoklonale Antikörper, Fragmente oder Derivate davon oder Aptamere wie in einem der vorstehenden Ansprüche definiert, gegebenenfalls fixiert an ein Trägermaterial.

47. Testvorrichtung zum Nachweis mindestens eines wie in einem der vorstehenden Ansprüchen definierten Epitops, umfassend
(a) mindestens zwei monoklonale Antikörper, Fragmente oder Derivate davon oder Aptamere wie in einem der vorstehenden Ansprüche definiert, fixiert an ein Trägermaterial;
(b) eine Vorrichtung zur Aufbereitung und Analyse von Stuhlproben; und gegebenfalls
(c) ein Gemisch von mindestens zwei monoklonalen Antikörpern, Fragmenten oder Derivaten davon oder Aptameren.

48. Testvorrichtung, zum Nachweis mindestens eines wie in einem der vorstehenden Ansprüchen definierten Epitops, umfassend
(a) mindestens zwei monoklonale Antikörper, Fragmente oder Derivate davon oder Aptamere wie in einem der vorstehenden Ansprüche definiert, wobei die Antikörper, Fragmente oder Derivate davon oder Aptamere konjugiert sind mit kolloidalem Gold, Latexpartikeln oder anderen farbgebenden Partikeln, deren Größe typischerweise im Bereich zwischen 5nm und 100nm, vorzugsweise zwischen 20nm und 60nm liegt;
(b) eine Vorrichtung zur Aufbereitung und Analyse von Stuhlproben; und gegebenfalls
(c) ein Gemisch von mindestens zwei monoklonalen Antikörpern, Fragmenten oder Derivaten davon oder Aptameren.

49. Kit enthaltend
(a) mindestens zwei monoklonale Antikörper, Fragmente oder Derivate davon oder Aptamere wie in einem der vorstehenden Ansprüche definiert, gegebenenfalls fixiert an ein Trägermaterial; gegebenenfalls
(b) eine Vorrichtung zur Aufbereitung und Analyse von Stuhlproben; und gegebenfalls
(c) ein Gemisch von mindestens zwei monoklonalen Antikörpern, Fragmenten oder Derivaten davon oder Aptameren.

50. Packung enthaltend die diagnostische Zusammensetzung nach Anspruch 46, die Testvorrichtung nach Anspruch 47, 48 oder den Kit nach Anspruch 49.

## Claims

1. A method for detecting an infection of an acid-resistant microorganism in a mammal, said method comprising
(a) incubating a stool sample of said mammal with two or three different monoclonal antibodies, fragments or derivatives thereof or aptamers under conditions allowing formation of a complex of antigens from the acid-resistant microorganisms with the antibodies, fragments or derivatives thereof or the aptamers and wherein
(aa) a first monoclonal antibody or a fragment or derivative thereof or a first aptamer specifically binds an epitope of a first antigen, which shows at least with some mammals a structure after the intestinal passage that corresponds to the native structure or to the structure which a mammal produces antibodies against after being infected or immunized with the acid-resistant microorganism or an extract or lysate thereof or a protein therefrom or a fragment thereof or a synthetic peptide comprising a sequence of the protein or fragment thereof,
(ab) a second monoclonal antibody or a fragment or derivative thereof or a second aptamer specifically binds an epitope of a second antigen different from the epitope of the first antigen, said epitope showing at least with a group of mammals a structure after the intestinal passage that corresponds to the native structure or the structure which a mammal produces antibodies against after being infected or immunized with the acid-resistant microorganism or an extract or lysate thereof or a protein therefrom or a fragment thereof or a synthetic peptide comprising a sequence of the protein or fragment thereof,
wherein the groups of mammals according to (aa) and (ab) may overlap and in total essentially make up more than 70% of the overall number of infected mammals; and
(b) detecting the formation of at least one antigen-antibody complex or antigen-aptamer complex according to (aa) or (ab).

2. The method according to claim 1, wherein the microorganism is an acid-resistant bacterium.

3. The method according to claim 2, wherein the acid-resistant bacterium is a bacterium belonging to the genus Helicobacter or the genus Mycobacterium or the genus Campylobacter.

4. The method according to claim 3, wherein the bacterium is a bacterium belonging to the species *Helicobacter pylori* or *Helicobacter hepaticus* or the species *Mycobacterium tuberculosis* or the species *Campylobacter jejuni* or *Campylobacter pylori.*

5. The method according to any one of claims 1 to 4, wherein the epitope of the first antigen is an epitope of a urease and the epitope of the second antigen is an epitope of a heat shock protein, an alkylhydroperoxide-reductase or of the 20kDa-protein (3-dehydro-quinase type II), the 16,9kDa-protein (neutrophil-activating protein) or the 33.8kDa protein (fructose-bisphosphate-aldolase).

6. The method according to claim 5, wherein the urease is β-urease of *H. pylori.*

7. The method according to one of claims 5 or 6, wherein the heat shock protein is a Hsp60.

8. The method according to any one of claims 5 to 7, wherein the alkylhydroperoxide-reductase is the 26kDa-protein of *H. pylori.*

9. The method according to any one of claims 1 to 8, said method comprising
(a) incubating a stool sample of a mammal with three different monoclonal antibodies, fragments or derivatives thereof or aptamers under conditions allowing the formation of a complex of antigens from the acid-resistant microorganism with said antibodies, fragments or derivatives or said aptamers and wherein
(aa) the first monoclonal antibody or the fragment or derivative thereof or the first aptamer specifically binds an epitope of the first antigen, which shows at least with a group of mammals a structure after the intestinal passage that corresponds to the native structure or the structure which a mammal produces antibodies against after being infected or immunised with the acid-resistant microorganism or an extract or lysate thereof or a protein therefrom or a fragment thereof or a synthetic peptide,
(ab) the second monoclonal antibody or the fragment or a derivative thereof or the second aptamer specifically binds an epitope of a second antigen different from the epitope of the first antigen, which shows at least with a group of mammals a structure after the intestinal passage that corresponds to the native structure or the structure which a mammal produces antibodies against after being infected or immunised with the acid-resistant microorganism or an extract or lysate thereof or a protein therefrom or a fragment thereof or a synthetic peptide, and
(ac) the third monoclonal antibody or the fragment or derivative or the third aptamer specifically binds an epitope of a third antigen different from the epitope of the first and second antigen, which shows at least with a group of mammals a structure after the intestinal passage that corresponds to the native structure or the structure which a mammal produces antibodies against after being infected or immunised with the acid-resistant microorganism or an extract or lysate thereof or a protein therefrom or a fragment thereof or a synthetic peptide,
wherein the groups of mammals according to (aa), (ab) and (ac) may overlap and in total essentially make up the overall number of infected mammals; and
(b) detecting the formation of at least one antigen-antibody complex or antigen-aptamer complex according to (aa), (ab) or (ac).

10. The method according to claim 9, wherein the epitope of the first antigen is an epitope of a urase, preferably of β-urease of *H. pylori,* the epitope of the second antigen is an epitope of a heat shock protein, preferably of Hsp60 of *H. pylori,* and the third antigen is an epitope of an alkylhydroperoxide-reductase, preferably of the 26kDa-protein of *H. pylori,* or wherein the epitope of the second and the third antigen is an epitope of a alkylhydroperoxide-reductase or of the 20kDa-protein (3-dehydro-quinase type II), of the 16,9kDa-protein (neutrophil-activating protein) or of the 33,8kDa-protein (fructose-bisphosphate-aldolase).

11. The method according to any one of claims 1 to 10, wherein at least one of the monoclonal antibodies or one of the fragments, derivatives or aptamers binds a conformational epitope.

12. The method according to claim 11, wherein all monoclonal antibodies or fragments, derivatives or aptamers bind conformational epitopes.

13. A method for detecting an infection with *Helicobacter pylori* in the stool of a mammal, the method comprising
(a) incubating a stool sample with two different monoclonal antibodies, fragments or derivatives thereof or aptamers under conditions allowing the formation of an antigen-antibody/antigen-aptamer complex, wherein
(aa) the first monoclonal antibody, the fragment, the derivative thereof or the first aptamer specifically binds β-urease or a fragment thereof;
(ab) the second monoclonal antibody, the fragment, the derivative thereof or the second aptamer specifically binds the 26kDa-antigen or a fragment thereof or specifically binds Hsp60 or a fragment thereof; and
(b) detecting the formation of at least one antigen-antibody complex/antigen-aptamer complex according to (aa) or (ab).

14. A method for detecting an infection with *Helicobacter pylori* in the stool of a mammal, said method comprising
(a) incubating a stool sample with at least three different monoclonal antibodies, fragments, derivatives thereof or aptamers under conditions allowing the formation of an antigen-antibody/antigen-aptamer complex, wherein
(aa) a first monoclonal antibody, a fragment, a derivative thereof or a first aptamer specifically binds β-urease or a fragment thereof;
(ab) a second monoclonal antibody, a fragment, a derivative thereof or a second aptamer specifically binds Hsp60 or a fragment thereof; and
(ac) a third monoclonal antibody, a fragment, a derivative thereof or a third aptamer specifically binds 26kDa-antigen or a fragment thereof; and
(b) detecting the formation of at least one antigen-antibody/antigen-aptamer complex according to (aa), (ab) or (ac).

15. The method according to any one of claims 7 to 14, wherein the Hsp60-specific antibody is an antibody which has been generated by hybridoma HP16m/2A5-E6-E5 deposited with the German Collection of Microorganisms and Cell Cultures (Deutsche Sammlung von Mikroorganismen und Zellkulturen DSMZ) on June 23, 1998 in accordance with the Statutes of the Budapest Treaty with the deposition number DSM ACC2356.

16. The method according to any one of claims 8 to 15, wherein the 26kDa-antigen-specific antibody is an antibody generated by hybridoma HP15m/3E8-D9-D6 deposited with the German Collection of Microorganisms and Cell Cultures (Deutsche Sammlung von Mikroorganismen und Zellkulturen DSMZ) on June 23, 1998 in accordance with the Statutes of the Budapest Treaty with the deposition number DSM ACC2355.

17. The method according to any one of claims 6 to 16, wherein the β-urease-specific antibody is an antibody generated by one of hybridomas HP8m/4H5-D4-C9 or HP9.1m/3C2-F8-E2 deposited with the German Collection of Microorganisms and Cell Cultures (Deutsche Sammlung von Mikroorganismen und Zellkulturen DSMZ) on June 23, 1998 in accordance with the Statutes of the Budapest Treaty with the deposition numbers DSM ACC2360 or DSM ACC2362.

18. The method according to any one of claims 7 to 17, wherein the heavy chain of the antibody binding an Hsp60-epitope comprises at least one of the following CDRs, preferably CDR3 and more preferably all of the following CDRs:
CDR1: GFSLSRYSVH
CDR2: MIWGGGSTDYNSGLKS
CDR3: NMGGRYPDYFDY

19. The method according to claim 18, wherein the DNA sequence encoding the heavy chain of the antibody is selected from at least one of the following DNA sequences encoding CDRs, preferably CDR3 and more preferably comprising all of the three following CDRs:
CDR1: GG GTTCTCATTA TCCAGATATA GTGTACAC
CDR2: ATGATATGGG GTGGTGGAAG CACAGACTAT AAT-TCAGGTC TCAAATCC
CDR3: AATATG GGGGGTAGGT ACCCGGACTA CTTTGACTAC

20. The method according to any one of claims 7 to 19, wherein the light chain of the antibody binding an Hsp60-epitope comprises at least one of the following CDRs, preferably CDR3 and more preferably all of the three following CDRs:
CDR1: RASKSVSTSGYSYIH
CDR2: LASNLES
CDR3: QHSRELPLT

21. The method according to claim 20, wherein the DNA sequence encoding the light chain of the antibody is selected from at least one of the following DNA sequences encoding CDRs, preferably CDR3 and more preferably comprising all of the three following CDRs:
CDR1: A GGGCCAGCAA GAGTGTCAGT ACATCTGGCT ATAGTTACAT ACAC
CDR2: C TTGCATCCAA CCTAGAATCT
CDR3: CAGC ACAGTAGGGA GCTTCCGCTC ACG.

22. The method according to any one of claims 8 to 21, wherein the heavy chain of the antibody binding a 26kDa-protein comprises at least one of the following CDRs, preferably CDR3 and more preferably all of the three following CDRs:
CDR1: GFTFNSYAMY
CDR2: RIRSKSDNYATYYANSVKD
CDR3: DHDKFPFYYALDY

23. The method according to claim 22, wherein the DNA sequence encoding the heavy chain of the antibody is selected from at least one of the following DNA sequences encoding CDRs, preferably CDR3 and more preferably comprising all of the three following CDRs:
CDR1: GG TTTCACCTTC AATTCCTATG CCATGTAC
CDR2: CGCATAAGAA GTAAAAGTGA TAATTATGCA ACATAT-TATG CCAATTCAGT GAAAGAC
CDR3: GATCATG ATAAGTTTCC TTTTTACTAT GCTCTGGACT AC

24. The method according to any one of claims 8 to 23, wherein an antibody, a fragment or a derivative thereof is used comprising the light chain of an antibody binding the 26kDa-protein comprising at least one of the following CDRs, preferably CDR3 and more preferably all of the three following CDRs:
CDR1: TASSSVSSSYLH
CDR2: STSNLAS
CDR3: HQYHRSPPT

25. The method according to claim 24, wherein the DNA sequence encoding the light chain of the antibody is selected from at least one of the following DNA sequences encoding CDRs, preferably CDR3 and more preferably comprising all of the three following CDRs:
CDR1: A CTGCCAGCTC AAGTGTGAGT TCCAGTTACT TGCAC
CDR2: AGCACTTCCA ACCTGGCTTC T
CDR3: CAC CAGTATCATC GTTCCCCACC GACG

26. The method according to any one of claims 6 to 25, wherein the heavy chain of the antibody binding the epitope of the β-urease comprises at least one of the following CDRs, preferably CDR3 and more preferably all of the three following CDRs:
CDR1: GFTFSSHFMS
CDR2: SISSGGDSFYPDSLKG
CDR3: DYSWYALDY
or:
CDR1: GYAFSTSWMN
CDR2: RIYPGDGDTNYNGKFKG
CDR3: EDAYYSNPYSLDY

27. The method according to claim 26, wherein the DNA sequence encoding the heavy chain of the antibody is selected from at least one of the following DNA sequences encoding CDRs, preferably CDR3 and more preferably comprising all of the three following CDRs:
CDR1: GG CTACGCATTC AGTACCTCCT GGATGAAC
CDR2: CGGATTTATC CTGGAGATGG AGATACTAAC TA-CAATGGGA AGTTCAAGGG C
CDR3: GAG GATGCCTATT ATAGTAACCC CTATAGTTTG GACTAC
or:
CDR1: GG ATTCACTTTC AGTAGCCATT TCATGTCT
CDR2: TCCATTAGTA GTGGTGGTGA CAGTTTCTAT CCA-GACAGTC TGAAGGGC
CDR3: GACTAC TCTTGGTATG CTTTGGACTA C

28. The method according to any one of claims 6 to 27, wherein the light chain of the antibody binding an epitope of the β-urease comprises at least one of the following CDRs, preferably CDR3 and more preferably all of the three following CDRs:
CDR1: RASQSIGTRIH
CDR2: YGSESIS
CDR3: QQSNTWPLT
or:
CDR1: HASQNINVWLS
CDR2: KASNLHT
CDR3: QQGRSYPLT

29. The method according to claim 28, wherein the DNA sequence encoding the light chain of the antibody is selected from at least one of the following DNA sequences encoding CDRs, preferably CDR3 and more preferably comprising all of the three following CDRs:
CDR1: A GGGCCAGTCA GAGCATTGGC ACAAGAATAC AC
CDR2: TAT GGTTCTGAGT CTATCTCT
CDR3: CAACAA AGTAATACCT GGCCGCTCAC G
or:
CDR1: C ATGCCAGTCA GAACATTAAT GTTTGGTTAA GC
CDR2: AAG GCTTCCAACT TGCACACA
CDR3: CAACAG GGTCGAAGTT ATCCTCTCAC G

30. The method according to any one of claims 6 to 29, wherein the variable regions of the light and heavy chains of the antibodies comprise the amino acid sequences depicted in Figures 1 and 2, Figures 3 and 4, Figures 5 and 6, or Figures 7 and 8, respectively.

31. The method according to any one of claims 6 to 30, wherein the coding regions of the variable regions of the light and heavy chains comprise the DNA sequences depicted in Figures 1 and 2, Figures 3 and 4, Figures 5 and 6, or Figures 7 and 8, respectively.

32. The method according to any one of claims 1 to 31, wherein the following steps are carried out with the stool sample before incubation with the antibodies: (a) resuspension of the stool sample in resuspension buffer in a ratio of 1:3 to 1:25, preferably approximately 1:10 and (b) mixing on a vortex mixer.

33. The method according to any one of claims 1 to 32, wherein the detection of the formation of the at least one antigen-antibody complex/antigen-aptamer complex in step (b) is carried out by means of an immunological method.

34. The method according to any one of claims 1 to 33, wherein the detection of the formation of the at least one antigen-antibody complex/antigen-aptamer complex in step (b) is carried out by means of ELISA, RIA, Western Blot or an immunochromatographic method.

35. The method according to claim 33 or 34, wherein RIA or ELISA is used for the detection and for RIA or ELISA the same type of antibody or a fragment or derivative thereof or the same type of aptamer is used for binding to the solid phase as well as for detecting an epitope.

36. The method according to any one of claims 1 to 35, wherein the antibodies, the fragments or derivatives thereof or the aptamers are fixed to a support.

37. The method according to any one of claims 1 to 36, wherein the monoclonal antibody is a mouse antibody.

38. The method according to claims 36, wherein the material of the support is a porous material.

39. The method according to claims 36 or 38, wherein the support is a test strip.

40. The method according to one of claims 36, 38 or 39, wherein the support is comprised of cellulose or a cellulose derivative.

41. The method according to any one of claims 1 to 40, wherein the mammal is a human.

42. A monoclonal antibody, fragment or derivative thereof which has a V region which comprises a combination of the CDRs described in any one of claims 18 to 29 or which has been generated by a hybridoma described in any one of claims 15 to 17.

43. The monoclonal antibody, fragment or derivative thereof according to claim 42 which has at least one of the V regions shown in Figures 1 to 8.

44. The monoclonal antibody, fragment or derivative thereof according to claims 42 or 43 which is a mouse antibody or a fragment or a derivative thereof or a chimeric, preferably humanized antibody or a fragment or derivative thereof.

45. An aptamer which specifically binds the same epitope as the monoclonal antibody, the fragment or derivative thereof according to any one of claims 42 to 44.

46. A diagnostic composition comprising at least two monoclonal antibodies, fragments or derivatives thereof or aptamers as defined in any one of the aforementioned claims, optionally fixed to a support.

47. A test device for the detection of at least one epitope as defined in any one of the aforementioned claims, comprising
(a) at least two monoclonal antibodies, fragments or derivatives thereof or aptamers as defined in any one of the aforementioned claims fixed to a support material;
(b) a device for preparing and analyzing stool samples and optionally
(c) a mixture of at least two monoclonal antibodies, fragments or derivatives thereof or aptamers.

48. A test device for the detection of at least one epitope as defined in any one of the aforementioned claims, comprising
(a) at least two monoclonal antibodies, fragments or derivatives thereof or aptamers as defined in any one of the aforementioned claims, wherein the antibodies, fragments or derivatives thereof or aptamers are conjugated with colloidal gold, latex particles or other coloring particles the size of which is typically between 5 nm and 100 nm, preferably between 20 nm and 60 nm
(b) a device for preparing and analyzing stool samples; and optionally
(c) a mixture of at least two monoclonal antibodies, fragments or derivatives thereof or aptamers.

49. A kit comprising
(a) at least two monoclonal antibodies, fragments or derivatives thereof or aptamers as defined in any one of the aforementioned claims, optionally fixed to a support material; optionally
(b) a device for preparing and analyzing stool samples; and optionally
(c) a mixture of at least two monoclonal antibodies, fragments or derivatives thereof or aptamers.

50. A package comprising the diagnostic composition according to claim 46, the test device according to claim 47 or 48 or the kit according to claim 49.

## Revendications

1. Procédé de détection ou de mise en évidence d'une infection d'un mammifère par un micro-organisme résistant aux acides, dans lequel
a) on fait incuber un échantillon de selles du mammifère avec deux ou trois anticorps monoclonaux différents, fragments ou dérivés de celui-ci ou aptamères, dans des conditions qui permettent la formation d'un complexe d'antigènes du micro-organisme résistant aux acides avec les anticorps, fragments ou dérivés de celui-ci ou les aptamères,
(aa) le premier anticorps monoclonal ou le fragment ou dérivé de celui-ci ou le premier aptamère se liant spécifiquement à un épitope du premier antigène qui présente au moins pour une partie des mammifères, après passage dans l'intestin, une structure qui correspond à la structure native ou à la structure contre laquelle un mammifère produit des anticorps après infection ou immunisation avec le micro-organisme résistant aux acides ou avec un extrait ou un lysat de celui-ci ou avec une protéine issue de celui-ci ou un fragment de celui-ci ou un peptide synthétique qui présente une séquence de la protéine ou du fragment ;
(ab) le deuxième anticorps monoclonal ou le fragment ou dérivé de celui-ci ou le deuxième aptamère se liant spécifiquement à un épitope différent de l'épitope du premier antigène d'un deuxième antigène qui présente au moins pour une partie des mammifères après passage dans l'intestin, une structure qui correspond à la structure native ou à la structure contre laquelle un mammifère produit des anticorps après infection ou immunisation avec le micro-organisme résistant aux acides ou avec un extrait ou un lysat de celui-ci ou avec une protéine issue de celui-ci ou un fragment de celui-ci ou un peptide synthétique qui présente une séquence de la protéine ou du fragment,
les parties de mammifères selon les points (aa) et (ab) pouvant se recouper et constituer au total plus de 70 % des mammifères infectés ; et
(b) on détecte la formation d'au moins un complexe antigène - anticorps ou un complexe antigène - aptamère selon le point (aa) ou le point (ab).

2. Procédé selon la revendication 1, dans lequel le micro-organisme est une bactérie résistante aux acides.

3. Procédé selon la revendication 2, dans lequel la bactérie résistante aux acides est une bactérie du genre *Helicobacter* ou du genre *Mycobacterium* ou du genre *Campylobacter*.

4. Procédé selon la revendication 3, dans lequel la bactérie est une bactérie de l'espèce *Helicobacter pylori* ou *Helicobacter hepaticus* ou de l'espèce *Mycobacterium tuberculosis* ou de l'espèce *Campylobacter jejuni* ou *Campylobacter pylori.*

5. Procédé selon l'une quelconque des revendications 1 à 4, dans lequel l'épitope du premier antigène est un épitope d'une uréase et l'épitope du deuxième antigène est un épitope d'une protéine de choc thermique, d'une alkylhydroxyperoxyde - réductase ou de la protéine de 20 kDa (3-déshydro-quinase de type II), de la protéine de 16,9 kDa (protéine d'activation des neutrophiles) ou de la protéine de 33,8 kDa (fructose-bisphosphate-aldolase).

6. Procédé selon la revendication 5, dans lequel l'uréase est la β-uréase de *H. pylori.*

7. Procédé selon la revendication 5 ou 6, la protéine de choc thermique étant une Hsp60.

8. Procédé selon l'une quelconque des revendications 5 à 7, dans lequel l'alkylhydroperoxyde-réductase est la protéine de 26 kDa de *H. pylori.*

9. Procédé selon l'une quelconque des revendications 1 à 8, dans lequel
a) on fait incuber un échantillon de selles du mammifère avec trois anticorps monoclonaux différents, fragments ou dérivés de celui-ci ou aptamères, dans des conditions qui permettent la formation d'un complexe d'antigènes du micro-organisme résistant aux acides avec les anticorps, fragments ou dérivés de celui-ci ou les aptamères,
aa) le premier anticorps monoclonal ou le fragment ou le dérivé de celui-ci ou le premier aptamère se liant spécifiquement à un épitope du premier antigène qui présente au moins pour une partie des mammifères, après passage dans l'intestin, une structure qui correspond à la structure native ou à la structure contre laquelle un mammifère produit des anticorps après infection ou immunisation avec le micro-organisme résistant aux acides ou avec un extrait ou un lysat de celui-ci ou avec une protéine issue de celui-ci ou un fragment de celui-ci ou un peptide synthétique ;
(ab) le deuxième anticorps monoclonal ou le fragment ou dérivé de celui-ci ou le deuxième aptamère se liant spécifiquement à un épitope différent de l'épitope du premier antigène d'un deuxième antigène qui présente au moins, pour une partie des mammifères, après passage dans l'intestin, une structure qui correspond à la structure native ou à la structure contre laquelle un mammifère produit des anticorps après infection ou immunisation avec le micro-organisme résistant aux acides ou avec un extrait ou un lysat de celui-ci ou avec une protéine issue de celui-ci ou un fragment de celui-ci ou un peptide synthétique ; et
(ac) le troisième anticorps monoclonal ou le fragment ou le dérivé de celui-ci ou le troisième aptamère se liant spécifiquement à un épitope différent de l'épitope du premier et du deuxième antigène d'un troisième antigène qui présente au moins, pour une partie des mammifères, après passage dans l'intestin, une structure qui correspond à la structure native ou à la structure contre laquelle un mammifère produit des anticorps après infection ou immunisation avec le micro-organisme résistant aux acides ou avec un extrait ou un lysat de celui-ci ou avec une protéine issue de celui-ci ou un fragment de celui-ci ou un peptide synthétique,
les parties de mammifères selon les points (aa) et (ab) et selon le point (ac) pouvant se recouper et constituant au total essentiellement la totalité des mammifères infectés ; et dans lequel
(b) on détecte la formation d'au moins un complexe antigène - anticorps ou d'un complexe antigène - aptamère selon le point (aa), (ab) ou le point (ac).

10. Procédé selon la revendication 9, dans lequel l'épitope du premier antigène est un épitope d'une uréase, de préférence de la β-uréase de *H. pylori*, l'épitope du deuxième antigène est un épitope d'une protéine de choc thermique, de préférence de Hsp60 de *H. pylori* et l'épitope du troisième anticorps est un épitope d'une alkylhydroperoxyde - réductase, de préférence de la protéine de 26 kDa de *H. pylori* ou dans lequel l'épitope du deuxième ou du troisième antigène est un épitope d'une alkylhydroperoxyde - réductase ou de la protéine de 20 kDa (3-déshydro-quinase de type II), de la protéine de 16,9 kDa (protéine d'activation des neutrophiles) ou de la protéine de 33,8 kDa (fructose-bisphosphate-aldolase).

11. Procédé selon l'une quelconque des revendications 1 à 10, dans lequel au moins l'un des anticorps monoclonaux ou l'un des fragments, dérivés ou aptamères se lie à un épitope conformationnel.

12. Procédé selon la revendication 11, dans lequel la totalité des anticorps monoclonaux ou fragments, dérivés ou aptamères se lie à des épitopes conformationnels.

13. Procédé de détection ou de mise en évidence d'une infection par *Helicobacter pylori* dans les selles d'un mammifère, dans lequel
(a) on fait incuber un échantillon de selles avec deux anticorps monoclonaux différents, fragments, dérivés de ceux-ci ou aptamères dans des conditions qui permettent la formation d'un complexe antigène - anticorps/antigène - aptamère,
(aa) le premier anticorps monoclonal, le fragment, dérivé de celui-ci ou le premier aptamère se liant spécifiquement à la β-uréase ou un fragment de celle-ci ;
(ab) le deuxième anticorps monoclonal, le fragment, dérivé de celui-ci ou le deuxième aptamère se liant spécifiquement à l'antigène de 26 kDa ou un fragment de celui-ci ou spécifiquement à la protéine Hsp60 ou un fragment de celle-ci ; et dans lequel
(b) on détecte la formation d'au moins un complexe antigène - anticorps/un complexe antigène - aptamère selon le point (aa) ou le point (ab).

14. Procédé de détection ou de mise en évidence d'une infection par *Helicobacter pylori* dans les selles d'un mammifère, dans lequel
(a) on fait incuber un échantillon de selles avec trois anticorps monoclonaux différents, fragments, dérivés de ceux-ci ou aptamères dans des conditions qui permettent la formation d'un complexe antigène - anticorps/antigène - aptamère,
(aa) le premier anticorps monoclonal, le fragment, dérivé de celui-ci ou le premier aptamère se liant spécifiquement à la β-uréase ou un fragment de celle-ci ;
(ab) le deuxième anticorps monoclonal, le fragment, dérivé de celui-ci ou le deuxième aptamère se liant spécifiquement à la protéine Hsp60 ou un fragment de celle-ci ; et
(ac) le troisième anticorps monoclonal, le fragment, dérivé de celui-ci ou le troisième aptamère se liant spécifiquement à l'antigène de 26 kDa ou un fragment de celui-ci ; et dans lequel
(b) on détecte la formation d'au moins un complexe antigène - anticorps/antigène - aptamère selon le point (aa), (ab) ou (ac).

15. Procédé selon l'une quelconque des revendications 7 à 14, dans lequel l'anticorps spécifique de Hsp60 est l'anticorps produit par l'hybridome HP16m/2A5-E6-E5 déposé auprès de la Deutsche Sammlung von Mikroorganismen und Zellkulturen (DSMZ) le 23 juin 1998 selon les statuts du traité de Budapest sous le numéro de dépôt DSM ACC2356.

16. Procédé selon l'une quelconque des revendications 8 à 15, dans lequel l'anticorps spécifique de l'antigène de 26 kDa est l'anticorps produit par l'hybridome HP15m/3E8-D9-D6 déposé auprès de la Deutsche Sammlung von Mikroorganismen und Zellkulturen (DSMZ) le 23 juin 1998 selon les statuts du traité de Budapest sous le numéro de dépôt DSM ACC2355.

17. Procédé selon l'une quelconque des revendications 6 à 16, dans lequel l'anticorps spécifique de la β-uréase est l'anticorps produit par l'hybridome HP8m/4H5-D4-C9 ou HP9.1m/3C2-F8-E2 déposé auprès de la Deutsche Sammlung von Mikroorganismen und Zellkulturen (DSMZ) le 23 juin 1998 selon les statuts du traité de Budapest sous le numéro de dépôt DSM ACC2360 ou DMS ACC2362.

18. Procédé selon l'une quelconque des revendications 7 à 17, dans lequel la chaîne lourde de l'anticorps se liant à l'épitope de Hsp60 présente au moins l'une des régions CDR suivantes, de préférence CDR3 et de manière davantage préférée, l'ensemble des trois CDR suivantes :
CDR1: GFSLSRYSVH
CDR2 : MIWGGGSTDYNSGLKS
CDR3 : NMGGRYPDYFDY

19. Procédé selon la revendication 18, dans lequel la séquence ADN codant pour la chaîne lourde de l'anticorps présente au moins l'une des séquences ADN suivantes codant pour les régions CDR, de préférence, la séquence ADN codant pour CDR3 et de manière davantage préférée, l'ensemble des trois séquences ADN suivantes codant pour les CDR :
CDR1 : GG GTTCTCATTA TCCAGATATA GTGTACAC
CDR2 : ATGATATGGG GTGGTGGAAG CACAGACTAT AATTCAGGTC TCAAATCC
CDR3: AATATG GGGGGTAGGT ACCCGGACTA CTTTGACTAC

20. Procédé selon l'une quelconque des revendications 7 à 19, dans lequel la chaîne légère de l'anticorps se liant à l'épitope de Hsp60 présente au moins l'une des régions CDR suivantes, de préférence CDR3 et de manière davantage préférée, l'ensemble des trois CDR suivantes :
CDR1: RASKSVSTSGYSYIH
CDR2: LASNLES
CDR3: QHSRELPLT

21. Procédé selon la revendication 20, dans lequel la séquence ADN codant pour la chaîne légère de l'anticorps présente au moins l'une des séquences ADN suivantes codant pour les régions CDR, de préférence, la séquence ADN codant pour CDR3 et de manière davantage préférée, l'ensemble des trois séquences ADN suivantes codant pour les CDR :
CDR1: A GGGCCAGCAA GAGTGTCAGT ACATCTGGCT ATAGTTACAT ACAC
CDR2 : C TTGCATCCAA CCTAGAATCT
CDR3 : CAGC ACAGTAGGGA GCTTCCGCTC ACG.

22. Procédé selon l'une quelconque des revendications 8 à 21, dans lequel la chaîne lourde de l'anticorps se liant à la protéine de 26 kDa présente au moins l'une des régions CDR suivantes, de préférence CDR3 et de manière davantage préférée, l'ensemble des trois CDR suivantes :
CDR1 : GFTFNSYAMY
CDR2 : RIRSKSDNYATYYANSVKD
CDR3: DHDKFPFYYALDY

23. Procédé selon la revendication 22, dans lequel la séquence ADN codant pour la chaîne lourde de l'anticorps présente au moins l'une des séquences ADN suivantes codant pour les régions CDR, de préférence, la séquence ADN codant pour CDR3 et de manière davantage préférée, l'ensemble des trois séquences ADN suivantes codant pour les CDR :
CDR1 : GG TTTCACCTTC AATTCCTATG CCATGTAC
CDR2: CGCATAAGAA GTAAAAGTGA TAATTATGCA ACATATTATG CCAATTCAGT GAAAGAC
CDR3: GATCATG ATAAGTTTCC TTTTTACTAT GCTCTGGACT AC.

24. Procédé selon l'une quelconque des revendications 8 à 23, dans lequel un anticorps, fragment ou dérivé de celui-ci est utilisé, dont la chaîne légère de l'anticorps se liant à une protéine de 26 kDa présente au moins l'une des régions CDR suivantes, de préférence CDR3 et de manière davantage préférée, l'ensemble des trois CDR suivantes :
CDR1 : TASSSVSSSYLH
CDR2 : STSNLAS
CDR3 : HQYHRSPPT.

25. Procédé selon la revendication 24, dans lequel la séquence ADN codant pour la chaîne légère de l'anticorps présente au moins l'une des séquences ADN suivantes codant pour les régions CDR, de préférence, la séquence ADN codant pour CDR3 et de manière davantage préférée, l'ensemble des trois séquences ADN suivantes codant pour les CDR :
CDR1: A CTGCCAGCTC AAGTGTGAGT TCCAGTTACT TGCAC
CDR2 : AGCACTTCCA ACCTGGCTTC T
CDR3 : CAC CAGTATCATC GTTCCCCACC GACG.

26. Procédé selon l'une quelconque des revendications 6 à 25, dans lequel la chaîne lourde de l'anticorps se liant à l'épitope de la β-uréase présente au moins l'une des régions CDR suivantes, de préférence CDR3 et de manière davantage préférée, l'ensemble des trois CDR suivantes :
CDR1: GFTFSSHFMS
CDR2 : SISSGGDSFYPDSLKG
CDR3 : DYSWYALDY
ou
CDR1: GYAFSTSWMN
CDR2 : RIYPGDGDTNYNGKFKG
CDR3 : EDAYYSNPYSLDY.

27. Procédé selon la revendication 26, dans lequel les séquences ADN codant pour la chaîne lourde de l'anticorps présentent au moins l'une des séquences ADN suivantes codant pour les régions CDR, de préférence, la séquence ADN codant pour CDR3 et de manière davantage préférée, l'ensemble des trois séquences ADN suivantes codant pour les CDR :
CDR1 : GG CTACGCATTC AGTACCTCCT GGATGAAC
CDR2: CGGATTTATC CTGGAGATGG AGATACTAAC TACAATGGGA AGTTCAAGGG C
CDR3: GAG GATGCCTATT ATAGTAACCC CTATAGTTTG GACTAC
ou
CDR1 : GG ATTCACTTTC AGTAGCCATT TCATGTCT
CDR2: TCCATTAGTA GTGGTGGTGA CAGTTTCTAT CCAGACAGTC TGAAGGGC
CDR3 : GACTAC TCTTGGTATG CTTTGGACTA C.

28. Procédé selon l'une quelconque des revendications 6 à 27, dans lequel la chaîne légère de l'anticorps se liant à l'épitope de la β-uréase présente au moins l'une des régions CDR suivantes, de préférence CDR3 et de manière davantage préférée, l'ensemble des trois CDR suivantes :
CDR1: RASQSIGTRIH
CDR2 : YGSESIS
CDR3 : QQSNTWPLT
ou
CDR1: HASQNINVWLS
CDR2: KASNLHT
CDR3 : QQGRSYPLT.

29. Procédé selon la revendication 28, dans lequel la séquence ADN codant pour la chaîne légère de l'anticorps présente au moins l'une des séquences ADN suivantes codant pour les régions CDR, de préférence, la séquence ADN codant pour CDR3 et de manière davantage préférée, l'ensemble des trois séquences ADN suivantes codant pour les CDR :
CDR1: A GGGCCAGTCA GAGCATTGGC ACAAGAATAC AC
CDR2 : TAT GGTTCTGAGT CTATCTCT
CDR3: CAACAA AGTAATACCT GGCCGCTCAC G
ou
CDR1: C ATGCCAGTCA GAACATTAAT GTTTGGTTAA GC
CDR2 : AAG GCTTCCAACT TGCACACA
CDR3 : CAACAG GGTCGAAGTT ATCCTCTCAC G.

30. Procédé selon l'une quelconque des revendications 6 à 29, dans lequel les anticorps dans les régions variables des chaînes légères et lourdes présentent les séquences d'acides aminés représentées sur les figures 1 et 2, les figures 3 et 4, les figures 5 et 6 ou les figures 7 et 8.

31. Procédé selon l'une quelconque des revendications 6 à 30, dans lequel les domaines codants des régions variables des chaînes légères et lourdes présentent les séquences ADN représentées sur les figures 1 et 2, les figures 3 et 4, les figures 5 et 6 ou les figures 7 et 8.

32. Procédé selon l'une quelconque des revendications 1 à 31, dans lequel les étapes suivantes sont réalisées avec l'échantillon de selles, avant incubation avec les anticorps : (a) remise en suspension de l'échantillon de selles selon un rapport 1:3 à 1:25, de préférence d'environ 1:10 dans un tampon de remise en suspension et (b) mélange dans un mélangeur vortex.

33. Procédé selon l'une quelconque des revendications 1 à 32, dans lequel la détection de la formation d'au moins un complexe antigène - anticorps /antigène - aptamère de l'étape (b) s'effectue au moyen d'un procédé immunologique.

34. Procédé selon l'une quelconque des revendications 1 à 33, dans lequel la détection de la formation d'au moins un complexe antigène - anticorps/antigène - aptamère de l'étape (b) s'effectue au moyen de la technique ELISA, d'un dosage radio-immunologique, du Western Blot ou par un procédé immuno-chromatographique.

35. Procédé selon la revendication 33 ou 34, dans lequel dans le procédé de dosage radio-immunologique ou d'ELISA, le même anticorps ou son fragment ou dérivé ou le même aptamère est utilisé pour liaison à la phase solide que pour la détection de l'épitope.

36. Procédé selon l'une quelconque des revendications 1 à 35, dans lequel les anticorps, fragments ou dérivés de celui-ci ou les aptamères sont fixés à un support.

37. Procédé selon l'une quelconque des revendications 1 à 36, dans lequel l'anticorps monoclonal est un anticorps de souris.

38. Procédé selon la revendication 36, dans lequel le matériau de support du support est un matériau de support poreux.

39. Procédé selon la revendication 36 ou 38, dans lequel le matériau de support est une bande de test.

40. Procédé selon la revendication 36, 38 ou 39, dans lequel le matériau de support est constitué de cellulose ou d'un dérivé de cellulose.

41. Procédé selon l'une quelconque des revendications 1 à 40, dans lequel le mammifère est un être humain.

42. Anticorps monoclonal, fragment ou dérivé de celui-ci, qui comporte une région V qui présente une combinaison de CDR présentées dans l'une quelconque des revendications 18 à 29 ou qui est produit par l'un des hybridomes représentés dans les revendications 15 à 17.

43. Anticorps monoclonal, fragment ou dérivé de celui-ci selon la revendication 42 qui présente au moins l'une des régions V représentée sur les figures 1 à 8.

44. Anticorps monoclonal, fragment ou dérivé de celui-ci selon la revendication 42 ou 43, qui est un anticorps de souris ou un fragment ou dérivé de celui-ci ou un anticorps chimère, de préférence humanisé ou un fragment ou dérivé de celui-ci.

45. Aptamère qui se lie spécifiquement au même épitope que l'anticorps monoclonal, le fragment ou dérivé de celui-ci selon l'une quelconque des revendications 42 à 44.

46. Composition diagnostique contenant au moins deux anticorps monoclonaux, fragments ou dérivés de ceux-ci ou aptamère tels que définis dans l'une quelconque des revendications précédentes, éventuellement fixés à un matériau de support.

47. Dispositif de test pour détecter ou mettre en évidence au moins un épitope tel que défini dans l'une quelconque des revendications précédentes, comprenant
(a) au moins deux anticorps monoclonaux, fragments ou dérivés de ceux-ci ou aptamères tels que définis dans l'une quelconque des revendications précédentes, fixés à un matériau de support ;
(b) un dispositif de préparation et d'analyse d'échantillons de selles ; et éventuellement
(c) un mélange d'au moins deux anticorps monoclonaux, fragments ou dérivés de ceux-ci ou aptamères.

48. Dispositif de test pour détecter ou mettre en évidence au moins un épitope tel que défini dans l'une quelconque des revendications précédentes, comprenant
(a) au moins deux anticorps monoclonaux, fragments ou dérivés de ceux-ci ou aptamères tels que définis dans l'une quelconque des revendications précédentes, dans lequel les anticorps, fragments ou dérivés de ceux-ci ou aptamères sont conjugués avec de l'or colloïdal, des particules de latex ou autres particules colorantes dont la taille se situe habituellement dans une plage de 5 nm à 100 nm, de préférence entre 20 nm et 60 nm ;
(b) un dispositif de préparation et d'analyse d'échantillons de selles ; et éventuellement
(c) un mélange d'au moins deux anticorps monoclonaux, fragments ou dérivés de ceux-ci ou aptamères.

49. Trousse ou ensemble contenant
a) au moins deux anticorps monoclonaux, fragments ou dérivés de ceux-ci ou aptamères tels que définis dans l'une quelconque des revendications précédentes, éventuellement fixés à un matériau de support ; éventuellement
(b) un dispositif de préparation et d'analyse d'échantillons de selles ; et éventuellement
(c) un mélange d'au moins deux anticorps monoclonaux, fragments ou dérivés de ceux-ci ou aptamères.

50. Conditionnement ou emballage contenant la composition diagnostique selon la revendication 46, le dispositif de test selon la revendication 47, 48 ou la trousse ou l'ensemble selon la revendication 49.
